(19) Europäisches Patentamt
European Patent Office
Office européen des brevets

(11) EP 0 396 459 B2

(12) **NOUVEAU FASCICULE DE BREVET EUROPEEN**

(45) Date de publication et mention de la décision concernant l'opposition:
**27.08.1997 Bulletin 1997/35**

(45) Mention de la délivrance du brevet:
**23.09.1992 Bulletin 1992/39**

(21) Numéro de dépôt: **90401162.4**

(22) Date de dépôt: **27.04.1990**

(51) Int. Cl.$^{6}$: **C01B 33/193**, A61K 7/16

(54) **Silice pour compositions dentifrices compatible notamment avec les composés organiques aminés**

Kieselsäure für Zahnpflegemittelmischungen, welche mit organischen, aminierten Verbindungen vereinbar sind

Silica for dentifrice compositions which is compatible with organic amino-compounds

(84) Etats contractants désignés:
**AT BE CH DE DK ES FR GB GR IT LI LU NL SE**

(30) Priorité: **03.05.1989 FR 8905868**

(43) Date de publication de la demande:
**07.11.1990 Bulletin 1990/45**

(73) Titulaire: **RHONE-POULENC CHIMIE**
**92408 Courbevoie Cédex (FR)**

(72) Inventeur: **Persello, Jacques**
**F-01120 La Boisse (FR)**

(74) Mandataire: **Dubruc, Philippe et al**
**RHONE-POULENC CHIMIE,**
**Direction de la Propriété Industrielle,**
**25, Quai Paul Doumer**
**92408 Courbevoie Cédex (FR)**

(56) Documents cités:
**EP-A- 0 157 703**
**EP-A- 0 315 503**
**DE-A- 2 344 805**
**US-A- 4 015 996**

- **"Schriftenreihe Pigmente" Nummer 9 der Degussa AG**
- **Precipitated Silicas and Silicates (Degussa)**



Printed by Rank Xerox (UK) Business Services
2.14.12/3.4

## Description

La présente invention concerne une silice utilisable notamment dans les compositions dentifrices, son procédé de préparation ainsi que dans compositions dentifrices comprenant cette silice.

On sait que la silice est couramment utilisée dans la préparation de compositions dentifrices. Elle peut y jouer d'ailleurs plusieurs rôles.

Elle agit tout d'abord comme agent abrasif en aidant par son action mécanique à l'élimination de la plaque dentaire.

Elle peut aussi jouer le rôle d'agent épaississant pour conférer des propriétés rhéologiques déterminées au dentifrice ainsi que d'agent optique pour lui donner la coloration souhaitée.

Par ailleurs, on sait que les dentifrices contiennent généralement une source de fluorure, le plus souvent le fluorure de sodium ou le monofluorophosphate utilisé comme agent anti-carie un agent liant par exemple, un colloïde d'algues comme la carraghénine, la gomme guar ou la gomme xanthane ; un agent humectant qui peut être un polyalcool, par exemple, la glycérine, le sorbitol, le xylitol et le propylèneglycol. On trouve également des constituants facultatifs, par exemple, des agents tensio-actifs, des agents pour diminuer la plaque dentaire ou le dépôt du tartre, des agents correcteurs du goût ; des agents colorants et pigments etc...

Parmi les différents composants de la formulation de dentifrice, on rencontre des composés organiques aminés. Par "composé organique aminé", on entend toute molécule active intervenant dans les formulations de dentifrice contenant au moins un atome d'azote. Plus spécifiquement, on peut citer les composés suivants :

- amines fluorées, utilisées comme agents anti-carie notamment les produits d'addition d'amines ou d'amino-acides à longue chaîne, avec le fluorure d'hydrogène, tels que l'hydrofluorure de cétylamine, le dihydrofluorure de bis-(hydroxyéthyl)aminopropyl N-hydroxyéthyl octadécylamine, le fluorure d'octadécylamine et le dihydrofluorure de N, N', N' tri-(polyoxyéthylene) N-hexadécylpropylènodiamine ;
- oxydes d'amines utilisés comme agents tensio-actifs non ioniques qui résultent de l'oxydation des amines aliphatiques tertiaires par l'eau oxygénée. On peut mentionner notamment les oxydes d'alkylamine de formule :

$$R(CH_3)_2\,N \rightarrow O$$

dans laquelle R est un radical alkyle linéaire ou ramifié ayant de 10 à 24 atomes de carbone environ.

On peut également citer des oxydes d'amines de formule :

$$R(CH_2CH_2OH)_2\,N \rightarrow O$$

- alkylamines qui peuvent être des amines aliphatiques primaires, secondaires, tertiaires ou quaternaires utilisées comme agents tensio-actifs cationiques. A titre d'exemples, on peut mentionner les alkylamines de formule $R-CN_2NH_2$ ou les diméthylalkylamines de formule $R-N(CH_3)_2$ ; le bromure de cétyltriméthylammonium.
- alkylbétaines qui sont des dérivés N-alkyl de la N-diméthylglycine et alkylamidoalkylbétaines désignées également par la suite par "alkylbétaines".

Comme exemples de cette classe de tensio-actifs amphotères, on peut donner :

. les alkylbétaines de formule :

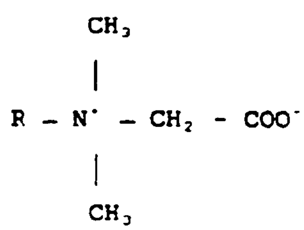

. les alkylamidopropyldiméthylbétaines de formule :

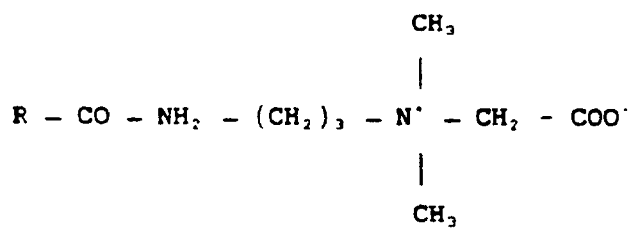

R étant un radical alkyle linéaire ou ramifié ayant de 10 à 24 atomes de carbone.

La présence de ces composés organiques aminés pose le problème de leur compatibilité avec la silice. En effet, à cause notamment de ses capacités adsorbantes, celle-ci peut avoir tendance à réagir avec lesdits composés de telle sorte qu'ils ne peuvent plus remplir la fonction qui leur est impartie.

L'objet de la présente invention est donc de fournir une nouvelle silice compatible notamment avec les composés organiques aminés précités plus particulièrement avec ceux de la classe des amines fluorées et des bétaïnes et donc parfaitement utilisables dans la formulation de dentifrice.

Un autre objet de l'invention est également de fournir une silice qui présente une bonne compatibilité vis-à-vis des différents cations présents dans la formulation de dentifrice, tels que par exemple zinc, strontium, étain, etc...

Un autre objet de l'invention est de fournir également une silice qui soit également la plus compatible avec les produits du type guanidines notamment les bis-biguanides dont l'élément le plus représentatif est la chlorhexidine.

Enfin, un autre objet de l'invention est le procédé de préparation de telles silices compatibles.

Or à cet effet, la Demanderesse s'est aperçu que les propriétés de compatibilité recherchées dépendaient essentiellement de la chimie de surface de la silice utilisée. La Demanderesse a ainsi établi un certain nombre de conditions sur la surface des silices pour que celles-ci soient compatibles.

La caractéristique de la silice de la présente invention est qu'elle conduit à une suspension aqueuse dont le pH varie en fonction de sa concentration dans l'aire définie par les deux inéquations :

$$- pH \leq 7.5 - 0,7 \log (C) \qquad (Ia)$$

et

$$- pH \geq 5,0 - 0,5 \log (C) \qquad (Ib)$$

et dont le pH varie en fonction de sa conductivité électrique dans l'aire définie par les deux inéquations :

$$- pH \leq 8,5 - 0,4 \log (D) \qquad (IIa)$$

et

$$- pH \geq 7,0 - 0,6 \log (D) \qquad (IIb)$$

- dans les inéquations (Ia) et (Ib),

  . (C) représente la concentration pondérale de la suspension aqueuse de silice exprimée en % de $SiO_2$,

- dans les inéquations (IIa) et (IIb),

  . (D) représente la conductivité électrique de la suspension aqueuse de silice exprimée en microsiemens.$cm^{-1}$.

Une autre caractéristique de la silice de l'invention est qu'elle présente une fonction d'acidité Ho d'au moins 4,0.

Une autre caractéristique de la silice de l'invention est qu'elle présente une nombre de sites $OH^-$ par $nm^2$ inférieur ou égal à 12.

Une autre caractéristique de la silice de l'invention est qu'elle présente un point de charge nulle (PZC) d'au moins 4.

Une caractéristique de la silice de l'invention est qu'elle présente une compatibilité d'au moins 30 % avec les composés organiques aminés et plus particulièrement d'au moins 50 % et, préférentiellement, d'au moins 80 % avec les composés organiques aminés choisis dans le groupe formé par les amines fluorées, les oxydes d'amines, les alkylamines et alkylbétaines.

Une autre caractéristique de la silice de l'invention est qu'elle présente une compatibilité d'au moins 50 %, et plus particulièrement d'au moins 70 % avec les cations métalliques.

Une autre caractéristique de la silice de l'invention selon une variante d'exécution est qu'elle présente une compatibilité avec les produits du type guanidine, notamment le chlorhexidine d'au moins 30 % et plus particulièrement d'au moins 60 %.

La présente invention a également pour objet l'un des procédés de préparation de la silice de l'invention qui est caractérisé par le fait qu'il consiste à faire réagir un silicate avec un acide conduisant ainsi à une suspension ou un gel de silice ; à effectuer un premier mûrissement à un pH supérieur ou égal à 6 et inférieur ou égal à 8,5 ; puis un deuxième mûrissement à un pH inférieur ou égal à 6.0 ; à effectuer un troisième mûrissement à un pH inférieur ou égal à 5,0 ; à séparer la silice ; à la soumettre à un lavage à l'eau jusqu'à ce qu'il conduise à une suspension aqueuse dont le pH mesuré sur une suspension à 20 % de $SiO_2$ réponde à l'équation suivante :

$$- pH = d - e \log (D) \qquad (III)$$

dans l'équation (III)

. e est une constante supérieure ou égale à 0,6 et inférieure ou égale à 1,0,
. d est une constante supérieure ou égale à 7,0 et inférieure ou égale à 8,5,
. (D) représente la conductivité électrique de la suspension aqueuse de silice exprimée en microsiemens.$cm^{-1}$ ; enfin à la sécher.

Enfin, l'invention concerne des compositions dentifrices caractérisées en ce qu'elles contiennent des silices telles que décrites ci-dessus ou préparées selon le procédé qui vient d'être mentionné plus haut

D'autres caractéristiques et avantages de l'invention seront mieux compris à la lecture de la description et des exemples concrets qui vont suivre.

La silice, objet de la présente invention, est caractérisée par la fait que le pH de sa suspension aqueuse varie en fonction de sa concentration et de sa conductivité électrique selon les équations données précédemment.

Le protocole de mesure du pH en fonction de la concentration de la suspension aqueuse de silice et de sa conductivité électrique est donnée, ci-après.

Comme cela a été indiqué en introduction, les caractéristiques essentielles des silices de l'invention résident dans leur chimie de surface. Plus précisément, un des aspects à pendre en compte dans cette chimie de surface est l'acidité. A ce sujet, l'une des caractéristiques des si ces de l'invention, est la force de leurs sites acides de surface.

Ici l'acidité est prise dans le sens de Lewis, c'est-à-dire qu'elle traduit la tendance d'un site à accepter une paire d'électrons d'une base selon l'équilibre :

$$B: + A \rightleftarrows BA$$

On utilise pour caractériser les silices de l'invention la notion de "fonction d'acidité" Ho, développée par Hammett, pour mesurer la tendance de l'acide, la silice dans le cas présent, à accepter une paire d'électrons d'une base.

La fonction Ho est ainsi définie par la relation classique :

$$pKa + \log \frac{(B:)}{(B:)(A)} = Ho$$

Pour déterminer la force des sites acides d'une silice de l'invention par la méthode de Hammett, on utilise la méthode des indicateurs décrite à l'origine par Walling (J. Am. Chem. Soc. 1950, <u>72</u>, 1164). La force des sites acides est déterminée par des indicateurs colorés dont on connaît, dans les conditions de l'emploi, le pKa de passage entre les formes acides et basiques.

Ainsi, plus bas est le pKa de l'indicateur subissant le changement de coloration, et plus forte est l'acidité du site. On a rassemblé dans le tableau ci-dessous, à titre d'exemple une liste, d'indicateurs de Hammett susceptibles d'être utilisés pour encadrer la valeur de Ho en déterminant sous qu'elle forme sont adsorbés deux indicateurs successifs.

| Indicateur | Couleur | | pKa |
|---|---|---|---|
| | Forme basique | Forme acide | |
| Neutral red | Jaune | Rouge | + 6,8 |
| Méthyl red | Jaune | Rouge | + 4,8 |
| Phénylazonaphthylamine | Jaune | Rouge | + 4,0 |
| p-Diméthylaminoazobenzène | Jaune | Rouge | + 3,3 |
| 2-Amino-5-azotoluène | Jaune | Rouge | + 2,0 |
| Benzèneazodiphénylamine | Jaune | Rouge | + 1,5 |
| 4-Diméthylaminoazo-1-naphtalène | Jaune | Rouge | + 1,2 |
| Crystal violet | Bleu | Jaune | + 0,8 |
| p-Nitrobenzèneazo-(p'-nitro)diphénylamine | Orange | Violet | + 0,43 |
| Dicinnamalacétone | Jaune | Rouge | - 3,0 |
| Benzalacétophénone | Incolore | Jaune | - 5,6 |
| Anthraquinone | Incolore | Jaune | - 8,2 |

La couleur des indicateurs adsorbés sur une silice est une mesure de la force des sites acides. Si la couleur est celle de la forme acide de l'indicateur, alors la valeur de la fonction Ho de la surface est égale ou inférieure au pKa de l'indicateur.

Des valeurs faibles de Ho, correspondent à des sites acides de force élevée.

Ainsi, par exemple, une silice donnant une coloration rouge avec le p-diméthylaminoazobenzène et jaune avec le 2-amino-5- azotoluène aura une fonction d'acidité Ho comprise entre 3,3 et 2.

Expérimentalement, le dosage s'effectue avec 0,2 g de silice placée dans un tube a essai en présence d'une solution d'indicateur à 100 mg/l dans le cyclohexane.

La silice est au préalable séchée à 190°C pendant 2 heures et conservée à l'abri de l'humidité dans un dessicateur.

Par agitation, l'adsorption, si elle a lieu, se produit en quelques minutes et on note le changement de couleur visible à l'oeil nu ou éventuellement en étudiant les spectres d'adsorption caractéristiques des indicateurs colorés adsorbés, aussi bien dans leur forme acide que dans leur forme basique.

La première caractéristique des silices de l'invention est qu'elles présentent une fonction d'acidité telle que déterminée ci-dessus d'au moins 4,0.

L'état de surface de la silice de l'invention est tel que des conditions sur le nombre de sites de surface sont remplies. Le nombre peut se mesurer en nombre de groupement $OH^-$ ou silanols par $nm^2$.

La détermination de ce nombre se fait de la manière suivante : le nombre de sites $OH^-$ de surface est assimilé à la quantité d'eau libérée par la silice entre 190°C et 900°C.

Les échantillons de silice sont préalablement séchés à 105°C pendant 2 heures.

Une masse $P_o$ de silice est placée dans une thermobalance et portée à 190°C pendant 2 heures : soit $P_{190}$, la masse obtenue.

La silice est portée ensuite à 900°C pendant 2 heures, soit $P_{900}$ la nouvelle masse obtenue.

Le nombre de sites $OH^-$ est calculé par l'équation suivante :

$$N_{OH^-} = \frac{66922,2}{A} \times \frac{P_{190} - P_{900}}{P_{190}}$$

dans laquelle :

- $N_{OH^-}$ est le nombre de sites $OH^-$ par $nm^2$ de surface
- A est la surface spécifique du solide mesurée par BET et exprimée en $m^2/g$.

Dans le cas présent, les silices de l'invention présentent avantageusement un nombre d'$OH^-/nm^2$ inférieur ou égal

à 12, plus particulièrement d'au plus 10 et notamment compris entre 6 et 10.

La nature des sites $OH^-$ des silices de l'invention qui constitue aussi une caractérisation de leur chimie de surface peut aussi être appréciée par le point de charge nulle.

Ce point de charge nulle (PZC) est défini par le pH d'une suspension de silice pour lequel la charge électrique de la surface du solide est nulle et cela quelle que soit la force ionique du milieu. Ce PZC mesure le pH réel de la surface, dans la mesure où celle-ci est libre de toutes impuretés de type ionique.

La charge électrique est déterminée par potentiomètrie. Le principe de la méthode est basé sur le bilan global des protons adsorbés ou désorbés sur la surface de la silice à un pH donné.

A partir des équations décrivant le bilan global de l'opération, il est facile de montrer que la charge électrique c de la surface, prise par rapport à une référence correspondant à une charge de surface nulle, est donnée par l'équation :

$$c = \frac{F}{A.M}(H^+) - (OH^-)$$

dans laquelle :

- A représente la surface spécifique du solide en $m^2/g$,
- M est la quantité de solide dans la suspension en g,
- F est le Faraday,
- $(H^+)$ ou $(OH^-)$ représente la variation par unité de surface de l'excès d'ions $H^+$ ou $OH^-$ respectivement sur le solide.

Le protocole expérimental de détermination du PZC est le suivant :

On utilise la méthode décrite par Berube et de Bruyn (J. Colloid Interface Sc. 1968, 27, 305).

La silice est lavée au préalable dans de l'eau désionisée de haute résistivité (10 Méga.Ohm.cm), séchée puis dégazée.

Pratiquement, on prépare une série de solutions à pHo = 8,5 par ajout de KOH ou $HNO_3$ et contenant un électrolyte indifférent ($KNO_3$) à une concentration variable entre $10^{-5}$ et $10^{-1}$ Mole/l.

A ces solutions, on ajoute une masse donnée de silice et on laisse le pH des suspensions obtenues se stabiliser sous agitation, à 25°C et sous azote pendant 24 heures ; soit pH'o sa valeur.

Des solutions étalons sont constituées par le surnageant obtenu par centrifugation pendant 30 mn à 1000 t/mn d'une partie de ces mêmes suspensions; soit pH'o, le pH de ces surnageants.

On ramène ensuite le pH d'un volume connu de ses suspensions et des solutions étalons correspondantes, à pHo en rajoutant la quantité nécessaire de KOH et on laisse les suspensions et les solutions étalons se stabiliser pendant 4 heures.

Soit $V_{OH-} . N_{OH-}$ le nombre d'équivalents de base ajoutée pour passer de pH'o à pHo d'un volume (V) connu de suspension ou de solution étalon.

Le dosage potentiométrique des suspensions et des solutions étalons est effectué à partir de pHo par addition d'acide nitrique jusqu'à pHf = 2,0.

Préférentiellement, on procède par addition d'incrément d'acide correspondant à une variation de pH de 0,2 unité de pH. Après chaque addition, le pH est stabilisé pendant 1 mn.

Soit $V_{H^+} . N_{H^+}$ le nombre d'équivalents d'acide pour parvenir à pHf.

A partir de pHo, on trace le terme ($V_{H^+} . N_{H^+} - V_{OH-} . N_{OH-}$) en fonction des pH incrémentés pour toutes les suspensions (3 forces ioniques au moins) et pour toutes les solutions étalons correspondantes.

Pour chaque valeur de pH (pas de 0,2 unité), on fait ensuite la différence entre la consommation de $H^+$ ou $OH^-$ pour la suspension et pour la solution étalon correspondante. On renouvelle cette opération pour toutes les forces ioniques.

Ceci donne le terme $(H^+) - (OH^-)$ correspondant à la consommation en protons de la surface. La charge de surface est calculée par l'équation ci-dessus.

On trace ensuite les courbes charge de surface en fonction du pH pour toutes les forces ioniques considérées. Le PZC est défini par l'intersection des courbes.

On ajuste la concentration en silice en fonction de la surface spécifique de celle-ci.

Par exemple, on utilise des suspensions à 2 % pour les silices de 50 $m^2/g$ à 3 forces ioniques (0,1 ; 0,01 et 0,001 mole/l).

Le dosage est effectué sur 100 ml de suspension en utilisant de l'hydroxyde de potassium à 0,1 M.

En pratique, il est préférable que la valeur de ce PZC soit d'au moins 4, plus particulièrement comprise entre 4 et 6. Dans le cas d'une meilleure compatibilité avec les cations métalliques, elle est d'au plus 6,5. Pour une bonne compatibilité avec le fluor, il est préférable d'avoir un PZC d'au plus 7.

Toujours pour l'amélioration de la compatibilité, notamment vis-à-vis du fluor, il est intéressant que la teneur en cations bivalents et plus contenus dans la silice soit au plus égale à 1000 ppm. Notamment, il est souhaitable que la teneur en aluminium des silices de l'invention soit au plus de 500 ppm.

D'autre part, la teneur en fer des silices de l'invention peut être avantageusement d'au plus 200 ppm.

Par ailleurs, d'une manière préférentielle, la teneur en calcium peut être d'au plus 500 ppm et plus particulièrement d'au plus 300 ppm.

Les silices de l'invention présentent aussi de préférence une teneur en carbone d'au plus 50 ppm et plus particulièrement d'au plus 10 ppm.

Les silices de l'invention qui sont compatibles avec les composés organiques aminés sont également compatible avec les différents cations métalliques intervenant dans les compositions de dentifrice.

En effet, celles-ci peuvent entre autres contenir des cations métalliques d'une valence supérieure à 1 apportés par les molécules actives. A titre d'exemples, on peut citer les cations métalliques bivalents et plus choisis dans les groupes 2a, 3a, 4a et 8 de la classification périodique. Plus précisément, on peut citer les cations du groupe 2a, calcium, strontium, baryum, les cations du groupe 3 a, aluminium, indium, du groupe 4a, germanium, étain, plomb et du groupe 8 : manganèse, fer, nickel, zinc, titane, zirconium, palladium, etc ...

Lesdits cations peuvent être sous forme de sels minéraux, par exemple chlorure, fluorure, nitrate, phosphate, sulfate ou sous forme de sels organiques acétate, citrate, etc...

Comme exemples plus spécifiques, on peut citer le citrate de zinc, le sulfate de zinc, le chlorure de strontium, le fluorure d'étain sous forme de sel simple ($SnF_2$) ou sous forme de sel double ($SnF_2$, KF), le chlorofluorure stanneux SnClF, le fluorure de zinc ($ZnF_2$).

Les silices de l'invention sont compatibles avec les différents cations métalliques. La compatibilité desdites silices avec les cations définie selon les tests décrits ci-après est d'au moins environ 50 %, de préférence d'au moins 70 % et encore plus préférentiellement d'au moins 80 %.

Les silices de l'invention peuvent donc être utilisées avantageusement dans les compositions de dentifrice contenant des cations bivalents et plus et, plus particulièrement, dans les compositions comprenant au moins l'un des composants suivants : citrate de zinc, sulfate de zinc, chlorure de strontium, fluorure d'étain.

Selon un mode particulier de l'invention, les silices de l'invention peuvent être compatibles en outre avec les guanidines et, notamment, avec la chlorhexidine. La compatibilité mesurée par le test décrit ci-après est d'au moins environ 30 %. Elle peut être améliorée et être d'au moins 60 % et, de préférence, d'au moins 90

Dans ce cas, la silice présente une teneuren anions du type $SO_4^{2-}$, $Cl^-$, $NO_3^-$, $PO_4^{3-}$, $CO_3^{2-}$ d'au plus $5.10^{-3}$ mole pour 100 g de silice. Cette compatibilité sera d'autant plus grande que cette teneur sera faible. Selon des variantes préférées elle sera d'au plus $1.10^{-3}$ moles, et plus particulièrement $0{,}2.10^{-3}$ moles pour 100 g de silice.

Dans le cas de silices préparées à partir d'acide sulfurique, on exprimera plus commodément cette teneur en anion par un teneur exprimée en $SO_4^{=}$ et en poids. Dans ce cas, cette teneur est d'au plus 0,1 %. Selon une variante préférée de l'invention, cette teneur est d'au plus 0,05 % et plus particulièrement d'au plus 0,01 %.

Les silices de l'invention sont donc particulièrement bien adaptées pour être mises en oeuvre dans des formulations de dentifrice contenant des guanidines, bisguanides. On peut mentionner ceux décrits dans le brevet US 3 934 002 ou 4 110 083.

Le pH des silices selon l'invention mesuré selon la norme NFT 45-007 est généralement au plus de 8. Il est plus particulièrement compris entre 6,0 et 7,5.

Les caractéristiques ci-dessus permettent d'avoir une silice compatible avec les composés organiques aminés précités, les cations métalliques et suivant le cas, avec en plus les fluorures et les guanidines, en particulier la chlorhexidine.

Outre les caractéristiques de chimie de surface qui viennent d'être décrites ci-dessus et qui conditionnent les compatibilités, les silices de l'invention présentent aussi des caractéristiques physiques qui les rendent parfaitement adaptées à leur application en dentifrice. Ces caractéristiques de type structurel vont être décrites ci-dessous.

Généralement la surface BET des silices de l'invention est comprise entre 40 et 600 $m^2/g$, plus particulièrement entre 40 et 350 $m^2/g$. Leur surface CTAB varie habituellement entre 40 et 400 $m^2/g$, plus particulièrement entre 40 et 200 $m^2/g$.

La surface BET est déterminée selon la méthode de BRUNAUER-EMMET-TELLER déorite dans the Journal of the American Chemical Society vol. 60, page 309, February 1938 et selon la norme NF X11-622 (3.3).

La surface CTAB est la surface externe déterminée selon la norme ASTM D3765 mais en pratiquant l'adsorption de bromure d'hexadécyltriméthyl ammonium (CTAB) à pH 9 et en prenant comme aire projetée de la molécule de CTAB 35 $Å^2$.

Les silices de l'invention peuvent bien entendu correspondre aux trois types habituellement distingués dans le domaine du dentifrice.

Ainsi, les silices de l'invention peuvent être du type abrasif. Elles présentent alors une surface BET comprise 40 et 300 $m^2/g$. Dans ce cas, la surface CTAB est comprise entre 40 et 100 $m^2/g$.

Les silices de l'invention peuvent aussi être du type épaississant. Elles ont alors une surface BET comprise entre 120 et 450 $m^2/g$, plus particulièrement 120 et 200 $m^2/g$. Elles pourront présenter alors une surface CTAB entre 120 et 400 $m^2/g$, plus particulièrement entre 120 et 200 $m^2/g$.

Enfin, selon un troisième type, les silices de l'invention peuvent être bifonctionnelles. Elles possèdent ici une surface BET comprise entre 80 et 200 $m^2/g$. La surface CTAB est alors comprise entre 80 et 200 $m^2/g$.

Les silices de l'invention peuvent aussi présenter une prise d'huile comprise entre 80 et 500 $cm^3/100$ g déterminée selon la norme NFT 30-022 (mars 53) en mettant en oeuvre le phtalate de dibutyle.

Plus précisément, cette prise d'huile sera comprise entre 100 et 140 $cm^3/100$ g pour les silices abrasives, 200 et 400 pour les silices épaississantes et 100 et 300 pour les bifonctionnnelles.

Par ailleurs, toujours en vue de l'application en dentifrice, les silices ont préférentiellement une taille de particules comprise entre 1 et 10 µm.

Cette taille moyenne ($d_{50}$) de particules est mesurée par Counter-Coulter.

La densité apparente variera généralement entre 0,01 et 0,3. Selon un mode de réalisation particulier de l'invention, les silices sont des silices de précipitation.

Enfin, les silices de l'invention présentent un indice de réfraction généralement compris entre 1,440 et 1,465.

Le procédé de préparation des silices de l'invention va maintenant être décrit plus particulièrement.

Comme indiqué plus haut, ce procédé est du type comprenant la réaction d'un silicate avec un acide ce qui donne lieu à la formation d'une suspension ou d'un gel de silice.

Il est à noter que l'on peut utiliser tout mode opératoire connu pour arriver à cette suspension ou ce gel, (addition d'acide sur un pied de cuve de silicate, addition simultanée totale ou partielle d'acide et de silicate sur un pied de cuve d'eau ou de solution de silicate etc...) le choix se faisant essentiellement en fonction des caractéristiques physiques de la silice que l'on désire obtenir.

Un mode de réalisation préférentiel de l'invention consiste à préparer la suspension ou le gel de silice en ajoutant simultanément le silicate et l'acide sur un pied de cuve qui peut être de l'eau, une dispersion colloïdale de silice contenant de 0 à 150 g/l de silice exprimée en $SiO_2$, un silicate ou un sel minéral ou organique, de préférence de métal alcalin tel que, par exemple, le sulfate de sodium, l'acétate de sodium.

L'addition des deux réactifs se fait de manière simultanée de telle sorte que le pH soit maintenu constant entre 4 et 10, de préférence entre 8,5 et 9,5. La température est avantageusement comprise entre 60°C et 95°C.

Un mode de préparation de la dispersion colloïdale de silice ayant de préférence une concentration entre 20 et 150 g/l consiste à chauffer une solution aqueuse de silicate par exemple entre 60°C et 95°C et à ajouter l'acide dans ladite solution aqueuse jusqu'à obtention d'un pH compris entre 8,0 et 10,0 de préférence aux environs de 9,5.

La concentration de la solution aqueuse de silicate exprimée en $SiO_2$ est de préférence comprise entre 20 et 150 g/l.

On peut faire appel à un acide dilué ou concentré : sa normalité pouvant varier entre 0,5 N et 36 N, de préférence entre 1 et 2 N.

Dans ce qui précède, on entend par silicate avantageusement un silicate de métal alcalin et, de préférence, un silicate de sodium de rapport pondéral $SiO_2/Na_2O$ compris entre 2 et 4, de préférence égal à 3,5. Pour ce qui est de l'acide, il peut être gazeux, comme le gaz carbonique ou liquide, de préférence, l'acide sulfurique.

Dans un autre étape du procédé de l'invention, on soumet la suspension ou le gel à des opérations de mûrissements.

On effectue un premier mûrissement à un pH d'au plus 8,5 et compris entre 6 et 8,5, par exemple, de préférence à 8,0. Le mûrissement se fait de préférence à chaud, par exemple à une température comprise entre 60°C et 100°C, de préférence 95°C et sur une durée qui peut varier entre 10 minutes et 2 heures.

Une autre variante d'exécution de l'invention consiste à préparer une suspension ou un gel de silice en ajoutant progressivement l'acide dans un pied de cuve contenant le silicate jusqu'à obtention du pH de mûrissement désiré. Cette opération est conduite à une température comprise, de préférence, entre 60°C et 95°C.

On effectue ensuite de mûrissement de la suspension du gel de silice dans les conditions décrites précédemment.

On réalise ensuite un deuxième mûrissement à un pH inférieur à 6, de préférence, compris entre 5 et 6 et, encore plus préférentiellement, égal à 5,5.

Les conditions de température et de durée sont celles du premier mûrissement.

A cet effet, on amène le pH au pH de mûrissement souhaité par ajout d'acide.

On peut faire appel, par exemple, à un acide minéral tel que l'acide nitrique, chlorhydrique, sulfurique, phosphorique ou bien à l'acide carbonique formé par barbotage de gaz carbonique.

On réalise un troisième mûrissement à un pH inférieur à 5,0, de préférence compris entre 3 et 5 et, encore plus préférentiellement, aux environs de 4,0.

Les conditions de température et de durée sont celles des deux autres mûrissements. On atteint le pH de mûrissement souhaité par ajout d'acide.

On procède alors à la séparation de la silice du milieu réactionnel selon tout moyen connu, filtre sous vide ou filtre presse par exemple.

On recueille ainsi un gâteau de silice.

Ensuite le procédé selon l'invention peut être mis en oeuvre selon deux variantes principales.

La première variante concerne la préparation de silices compatibles avec les composés organiques aminés et les cations métalliques bivalents et plus.

Dans ce cas, le procédé comporte un lavage du gâteau qui est réalisé dans des conditions telles que le pH de la

suspension ou du milieu avant séchage doit répondre à l'équation suivante :

$$- pH = d - e \log (D) \qquad (III)$$

dans l'équation (III)

. e est une constante supérieure ou égale à 0,6 et inférieure ou égale à 1,0,
. d est une constante supérieure ou égale à 7,0 et inférieure ou égale à 8,5,
. (D) représente la conductivité électrique de la suspension aqueuse de silice exprimée en microsiemens.$cm^{-1}$.

A cet effet, on peut envisage de faire un lavage à l'eau, généralement désionisée et/ou un lavage à l'aide d'une solution acide ayant un pH compris entre 2 et 7.

Cette solution acide peut être par exemple une solution d'un acide minéral tel que l'acide nitrique.
Toutefois selon un mode de réalisation particulier de l'invention, cette solution acide peut aussi être une solution d'un acide organique notamment d'un acide organique complexant. Cet acide pourra être choisi dans le groupe des acides carboxyliques, dicarboxyliques, hydroxycarboxyliques et amino-carboxyliques.
On peut citer comme exemple de tels acides l'acide acétique et pour les acides complexants, l'acide tartrique, l'acide maléique, l'acide glycérique, l'acide gluconique, l'acide citrique.
Il peut être avantageux, surtout dans le cas de l'emploi d'une solution d'un acide minéral, de procéder à un ultime lavage par de l'eau désionisée.

La deuxième variante se rapporte à la préparation de silices qui soient en outre compatibles avec les guanidines et, notamment, la chlorhexidine.

En pareil cas, on effectue un lavage plus poussé. Il doit être poursuivi jusqu'à ce que l'on obtienne un filtrat de lavage dont la conductivité soit d'au plus 200 microsiemens.$cm^{-1}$, de préférence inférieure à 100 microsiemens.$cm^{-1}$.
Comme mentionné précédemment, il importe que la concentration en anions soit d'au plus $5.10^{-3}$ mole pour 100 g de silice.
On peut effectuer, selon le cas, un ou plusieurs lavages, généralement deux à l'eau, de préférence désionisée et/ou à l'aide d'une solution aqueuse d'un acide organique, notamment ceux précités.

D'un point de vue pratique, les opérations de lavage peuvent se faire par passage de la solution de lavage sur le gâteau ou par introduction de celle-ci dans la suspension obtenue, après délitage du gâteau.
En effet le gâteau de filtration est soumis avant l'opération de séchage à un délitage qui peut être effectué par tout moyen connu, par exemple, à l'aide d'un agitateur tournant à grande vitesse.

Le gâteau de silice, avant ou après lavage, est donc délité puis séché selon tout moyen connu. Le séchage peut se faire par exemple, dans un four tunnel ou à moufle ou par atomisation dans un courant d'air chaud dont la température d'entrée peut varier entre 200°C et 500°C environ et la température de sortie entre 80°C et 100°C environ : le temps de séjour étant compris entre 10 secondes et 5 minutes.

Le produit séché peut être broyé si nécessaire pour obtenir la granulométrie désirée. L'opération est conduite dans un appareillage classique : broyeur à couteaux ou broyeur à jet d'air.

L'invention concerne aussi des compositions dentifrices contenant les silices du type décrit ci-dessus ou obtenues par le procédé qui vient d'être étudié.

La quantité de silice selon l'invention utilisée dans les compositions dentifrices peut varier dans le larges limites : elle est habituellement comprise entre 5 et 35 %.

Les silices de l'invention s'appliquent particulièrement bien aux compositions dentifrices contenant au moins un élément choisi dans le groupe comprenant les fluorures, les phosphates, les guanidines, dont la chlorhexidine. Elles peuvent en effet présenter une compatibilité selon les tests décrits ci-après d'au moins 90 % pour chacun de ses éléments.

Elles conviennent également bien aux compositions dentifrice contenant au moins un élément choisi dans le groupe comprenant les composants organiques aminés et les composants apportant un cation métallique bivalent et plus. Elles peuvent alors présenter une compatibilité selon les tests décrits ci-après qui peut atteindre 80 % pour chaque élément.

Les silices de l'invention sont particulièrement bien adaptées aux formulations de dentifrice comprenant simultanément au moins un élément choisi dans le groupe des fluorures minéraux simples et des fluorures organiques, au moins un élément choisi dans le groupe des alkylbétaines et au moins un élément choisi dans le groupe des guanidines, en particuler la chlorhexidine.

On peut mentionner plus précisément les formulations comprenant un fluorure de sodium et/ou un fluorure d'étain et/ou une amine fluorée notamment l'hydrofluorure de cétylamine, le dihydrofluorure de bis(hydroxyéthyl)-aminopropyl-N-hydroxyéthyl-octadécylamine et une alkylbétaine et de la chlorhexidine.

Les silices de l'invention sont en outre compatibles avec les copolymères acide maléique-vinyléthyléther et peuvent donc aussi être incorporées à des compositions dentifrices comprenant ces copolymères.

En ce qui concerne les composés fluorés, leur quantité correspond de préférence à une concentration en fluor dans la composition comprise entre 0,01 et 1 % en poids et plus particulièrement 0,1 % à 0,5 %. Les composés fluorés sont en particulier les sels de l'acide monofluorophosphorique et particulièrement ceux de sodium, potassium, lithium, calcium, aluminium et ammonium, le mono et le difluorophosphate ainsi que des fluorures variés contenant le fluor sous forme d'ion lié particulièrement les fluorures alcalins comme ceux de sodium, lithium, potassium, le fluorure d'ammonium, le fluorure stanneux, le fluorure de manganèse, le fluorure de zirconium, le fluorure d'aluminium ainsi que des produits d'addition de ces fluorures entre eux ou avec d'autres fluorures, tels que les fluorures de potassium ou de sodium ou de manganèse.

D'autres fluorures sont également utilisables pour la présente invention comme, par exemple, le fluorure de zinc, le fluorure de germanium, le fluorure de palladium, le fluorure de titane, les fluozirconates alcalins par exemple de sodium ou de potassium, le fluozirconate stanneux, le fluoborate ou les fluosulfates de sodium, de potassium.

Les composés fluorés organiques mentionnés au début de la description peuvent également être utilisés, de préférence le fluorure de cétylamine et le dihydrofluorure de bis-(hydroxyéthyl)aminopropyl N-hydroxyéthyl octadécylamine.

En ce qui concerne les composants apportant les cations métalliques bivalents et plus, parmi ceux précités dans la description, les plus couramment rencontrés sont le citrate de zinc, le sulfate de zinc, le chlorure de strontium, le fluorure d'étain.

Pour les éléments utilisables comme agents anti-plaques du type polyphosphates ou polyphosphonates, guanidines, bis-biguanides on peut mentionner ceux indiqués dans les brevets US 3.934.002 ou 4.110.083.

Les compositions dentifrices peuvent contenir en outre un liant.

Les principaux liants utilisés sont notamment choisis parmi :

- les dérivés cellulosiques : méthylcellulose, hydroxyéthylcellulose, carboxyméthylcellulose sodique,
- les mucilages : carraghénates, alginates, agar-agar et géloses,
- les gommes : gommes arabique et adragante, gomme xanthane, gomme karaya,
- les polymères carboxyvinyliques et acryliques,
- les résines de polyoxyéthylène.

Outre les silices de l'invention, les compositions dentifrices peuvent contenir aussi un ou plusieurs autres agents abrasifs polissants choisis notamment parmi :

- le carbonate de calcium précipité,
- le carbonate de magnésium,
- les phosphates de calcium, di- et tricalciques,
- le métaphosphate de sodium insoluble,
- le pyrophosphate de calcium,
- l'oxyde de titane (agent de blanchiment),
- les silicates,
- les alumines et silico-aluminates,
- les oxydes de zinc et d'étain,
- le talc,
- le kaolin.

Les compositions dentifrices peuvent aussi comprendre des tensio-actifs, des humectants, des agents aromatisants, édulcorants et des colorants et des conservateurs.

Les principaux tensio-actifs utilisés sont notamment choisis parmi :

- le laurylsulfate de sodium,
- le lauryléthersulfate et le laurylsulfoacétate de sodium,
- le dioctylsulfosuccinate de sodium,
- le laurylsarcosinate de sodium,
- le ricinoléate de sodium,
- les monoglycérides sulfatés.

Les principaux agents humectants utilisés sont notamment choisis parmi les polyalcools comme :

- le glycérol,
- le sorbitol, généralement en solution à 70 % dans l'eau,
- le propylène glycol.

Les principaux agents aromatisants (parfum) sont notamment choisis parmi : les essences d'anis, de badiane, de menthe, de baie de genièvre, de cannelle, de girofle et de rose.

Les principaux agents édulcorants sont notamment choisis parmi les imides orthosulfobenzoïques et le cyclamates.

Les principaux colorants utilisés sont notamment choisis selon la couleur désirée parmi :

- coloration rouge et rose : amaranthe, azorubine, cachou, coccine nouvelle (PONCEAU 4 R), cochenille, erythrosine,
- coloration verte : chlorophylle et chlorophylline,
- coloration jaune : jaune soleil (Orange S) et jaune de quinoléine.

Les principaux conservateurs les plus utilisés sont : les parahydroxybenzoates, le formol et les produits qui en dégagent, l'héxétidine, les ammoniums quaternaires, l'hexachlorophène, le bromophène et l'hexamédine.

Enfin, les compositions dentifrices contiennent des agents thérapeutiques dont les principaux sont notamment choisis parmi :

- les antiseptiques et les antibiotiques,
- les enzymes,
- les oligo-éléments et les composés fluorés qui ont été décrits ci-dessus.

Des exemples concrets de l'invention vont maintenant être donnés.

Auparavant, le protocole de mesure de pH en fonction de la conductivité et de la concentration ainsi que les tests pour la mesure de la compatibilité de la silice avec différents éléments vont être décrits.

PROTOCOLE DE MESURE DU pH EN FONCTION DE LA CONCENTRATION EN SILICE ET DE LA CONDUCTIVITE

Des suspensions de silice de concentration croissante variant de 0 à 25 % en poids sont constituées en dispersant une masse m de silice préalablement séchées à 120°C pendant 2 heures dans une masse 100-m d'eau désionisée et dégazée (Qualité Millipore). Les suspensions sont agitées pendant 24 heures à 25°C.

Le pH des suspensions et des solutions obtenues après centrifugation d'une partie de la suspension à 8000 tours/mn pendant 40 mn et filtration sur filtre Millipore 0,22 µm, sont mesurés à 25°C sous atmosphère d'azote avec un système de mesure de type Titroprocessor Metrohm 672.

De la même façon, on mesure la conductivité des suspensions et des solutions obtenues comme précédemment à 25°C avec un conductivimètre Radiometer (CDM83) équipé d'une cellule de type CDC304 de constante de cellule égale à 1 cm-1. La conductivité est exprimée en µSiemens/cm.

L'effet de suspension (SE) est défini par la différence de pH entre le pH d'une suspension à 20% en silice et le pH de sa solution surnageante séparée par centrifugation.

Mesure de la compatibilité avec le dihydrofluorure de bis (hydroxyéthyl)-aminopropyl-N-hydroxyéthyl-octadécylamine

1) Une solution aqueuse (1) contenant 1,65 % d'amine fluorée est constituée en ajoutant 5 g de solution d'amine fluorée à 33 % dans le propanediol dans 95 g d'eau bi-distillée.

2) On disperse 4 g de silice dans 16 g de solution obtenue en 1).

La suspension ainsi obtenue est agitée pendant 4 semaines à 37°C.

3) La suspension est ensuite centrifugée à 8000 tours/mn pendant 30 mn et le surnageant obtenu (3) est filtré sur filtre Millipore 0,22 µm.

4) La concentration en amine fluorée libre est déterminée par microanalyse de l'azote dans la solution obtenue en 1) et dans le surnageant obtenu en 3).

5) La compatibilité est déterminée par la relation ci-dessous :

$$\% \text{ compatibilité} = \frac{\text{concentration en N dans le surnageant(3)}}{\text{concentration en N dans la solution(1)}} \times 100$$

Par la suite, % Compatibilité en amine fluorée est désigné par AF.

Mesure de la compatibilité avec l'hydrofluorure de cétylamine

1) Une solution aqueuse (1) contenant 1,72 % d'amine fluorée est constituée en dissolvant 1,72 g d'hydrofluorure de cétylamine dans 98,28 g d'eau bi-distillée.

2) On disperse 4 g de silice dans 16 g de solution obtenue en 1).

La suspension ainsi obtenue est agitée pendant 4 semaines à 37°C.

3) La suspension est ensuite centrifugée à 8000 tours/mn pendant 30 mn et le surnageant obtenu (3) est filtré sur filtre Millipore 0,22 µm.

4) La concentration en amine fluorée libre est déterminée par microanalyse de l'azote dans la solution obtenue en 1) et dans le surnageant obtenu en 3).

5) La compatibilité est déterminée par la relation ci-dessous :

$$\% \text{ compatibilité} = \frac{\text{concentration en N dans le surnageant(3)}}{\text{concentration en N dans la solution(1)}} \times 100$$

Par la suite, % Compatibilité en amine fluorée est désigné par $AF_C$.

Mesure de la compatibilité avec une alkylbétaine

L'alkylbétaine mise en oeuvre est un produit commercialisé par AKSO sous la marque ARMOTERIC LB.

1) Une solution aqueuse (1) contenant 2,0 % d'alkylbétaine est constituée en dissolvant 6,67 g d'alkylbétaine à 30 % dans 93,33 g d'eau bi-distillée.

2) On disperse 4 g de silice dans 16 g de solution obtenue en 1).

La suspension ainsi obtenue est agitée pendant 4 semaines à 37°C.

3) La suspension est ensuite centrifugée à 8000 tours/mn pendant 30 mn et le surnageant obtenu (3) est filtré sur filtre Millipore 0,22 µm.

4) La concentration en alkylbétaine libre est déterminée par microanalyse du carbone organique dans la solution obtenue en 1) et dans le surnageant obtenu en 3).

5) La compatibilité est déterminée par la relation ci-dessous :

$$\% \text{ compatibilité} = \frac{\text{concentration en C dans le surnageant(3)}}{\text{concentration en C dans la solution(1)}} \times 100$$

Par la suite, % Compatibilité alkylbétaine est désigné par aBéta.

Mesure de la compatibilité avec une alkylamidoalkylbétaine

1) Une solution aqueuse (1) contenant 2,0 % d'alkylamidoalkylbétaine est constituée en dissolvant 6,67 g d'alkylamidoalkylbétaine à 30 % dans 93,33 g d'eau bi-distillée.

2) On disperse 4 g de silice dans 16 g de solution obtenue en 1).

La suspension ainsi obtenue est agitée pendant 4 semaines à 37°C.

3) La suspension est ensuite centrifugée à 8000 tours/nm pendant 30 mn et le surnageant obtenu (3) est filtré sur filtre Millipore 0,22 µm.

4) La concentration en alkylamidoalkylbétaine libre est déterminée par microanalyse du carbone organique dans la solution obtenue en 1) et dans le surnageant obtenu en 3).

5) La compatibilité est déterminée par la relation ci-dessous :

$$\% \text{ compatibilité} = \frac{\text{concentration en C dans le surnageant(3)}}{\text{concentration en C dans la solution(1)}} \times 100$$

Par la suite, % Compatibilité alkylamidoalkylbétaine est désigné par Béta.

Mesure de la compatibilité avec la chlorhexidine

4 g de silice sont dispersés dans 16 g d'une solution aqueuse de chlorhexidine de concentration 1 % en digluconate de chlorhexidine.

La suspension est agitée pendant 24 heures à 37°C.

La suspension est ensuite centrifugée à 20000 t/mn pendant 30 mn et le surnageant obtenu est filtré sur filtre Millipore 0,2 µm.

Par la suite 0,5 ml de solution ainsi filtrée est prélevé et dilué dans 100 ml d'eau dans une fiole jaugée. Cette solution constitue la solution de l'essai.

Une solution de référence est constituée selon le même protocole mais en absence de silice. Une solution aqueuse

à 1 % de digluconate de chlorhexidine est agitée pendant 24 heures à 37°C, puis est centrifugée à 20000 t/mn et le surnageant est filtré sur filtre Millipore de 0,2 µm. 0,5 ml de solution ainsi obtenue est dilué dans 100 ml d'eau dans une fiole jaugée.

On mesure ensuite l'absorbance des deux solutions à 254 nm à l'aide d'un spectrophotomètre (Uvikon 810/820).

La quantité de chlorhexidine libre notée % Compatibilité, est déterminée par le rapport :

$$\% \text{ compatibilité} = \frac{\text{Absorbance de l'essai}}{\text{Absorbance de la référence}} \times 100$$

Mesure de la compatibilité avec les fluorures

4 g de silice sont dispersés dans 16 g de solution à 0,3 % de fluorure de sodium (NaF). La suspension est agitée pendant 24 heures à 37°C. Après centrifugation de la suspension à 20000 t/mn pendant 30 mn, le surnageant est filtré sur filter Millipore 0,2 µm. La solution ainsi obtenue, constitue la solution de l'essai.

Une solution de référence est constituée en utilisant le même protocole mais en absence de silice.

La compatibilité avec les fluorures est déterminée parle % de fluorure libre mesuré parélectrode sélective à fluorure (Orion). Elle est déterminée par la relation ci-dessous.

$$\% \text{ compatibilité} = \frac{\text{Concentration en F de l'essai (ppm)}}{\text{Concentration en F de la référence (ppm)}} \times 100$$

Mesure de la compatibilité avec le zinc

4 g de silice sont dispersés dans 100 ml de solution à 0,06 % de $ZnSO_4$, $7H_2O$. On obtient une suspension dont le pH est stabilisé à 7 pendant 15 minutes par ajout de NaOH ou $H_2SO_4$. La suspension est agitée ensuite pendant 24 heures à 37°C puis est centrifugée à 20000 t/mn pendant 30 mn.

Le surnageant filtré sur filtre Millipore 0,2 µm, constitue la solution de l'essai.

Une solution de référence est constituée en suivant le même protocole mais en absence de silice.

La concentration en zinc libre des deux solutions est déterminée par absorption atomique (214 nm).

La compatibilité est déterminée par la relation ci-dessous :

$$\% \text{ compatibilité} = \frac{\text{Concentration en Zn de l'essai (ppm)}}{\text{Concentration en Zn de la référence (ppm)}} \times 100$$

Mesure de la compatibilité avec les pyrophosphates de sodium et de potassium

4 g de silice sont dispersés dans 16 g de suspension à 1,5 % de pyrophosphate de sodium ou de potassium. La suspension est agitée pendant 24 heures à 37°C puis est centrifugée à 20000 t/mn pendant 30 mn.

Le surnageant est filtré sur filtre Millipore 0,2 µm. 0,2 g de solution diluée dans 100 ml d'eau dans une fiole jaugée, constitue la solution de l'essai.

Une solution de référence est constituée en suivant le même protocole mais en absence de silice.

La concentration en ion pyrophosphate ($P_2O_7^{--}$) libre des deux solutions est déterminée par chromatographie ionique (système DIONEX 2000i) équipé d'un intégrateur.

La compatibilité est déterminée par le rapport des aires des pics obtenus sur les chromatogrammes et correspondant au temps de rétention du pyrophosphate, de l'essai et de la référence.

$$\% \text{ compatibilité} = \frac{\text{Aire du pic de l'essai}}{\text{Aire du pic de la référence}} \times 100$$

EXEMPLE 1

Dans un réacteur équipé d'un système de régulation de température et de pH et d'un système d'agitation à hélice (Mixel), on introduit 8,32 l de silicate de sodium de concentration en silice de 130 g/l et de rapport molaire $SiO_2/Na_2O$ = 3,5 et 8,33 l d'eau permutée de conductivité 1 µS/cm.

Après la mise en marche de l'agitation (350 t/mn), le pied de cuve ainsi constitué est chauffé à 90°C.

Lorsque la température est atteinte, on procède à l'addition d'acide sulfurique de concentration 80 g/l avec un débit constant de 0,40 l/mn pour amener le pH à 9,5.

On procède par la suite à l'addition simultanée de 45,25 l de silicate de sodium de concentration en silice de 130 g/l, de rapport molaire $SiO_2/Na_2O$ = 3,5 et de débit égal à 0,754 l/mn et de 29,64 l d'acide sulfurique à 80 g/l. Le débit d'acide sulfurique et ajusté de manière à maintenir le pH du milieu réactionnel à une valeur constante de 9,5.

Après 60 mn d'addition, on arrête l'addition de silicate de sodium et on continue l'ajout d'acide sulfurique avec un débit de 0,494 l/mn jusqu'à stabilisation du pH du mélange réactionnel à 8,0. Pendant cette phase, la température du milieu est portée à 95°C. On réalise, par la suite, un mûrissement de 30 mn à ce pH et à 95°C. Pendant le mûrissement, le pH est maintenu à 8 par ajout d'acide.

En fin de mûrissement, le pH est amené à 5,5 par ajout d'acide sulfurique avec un débit de 0,494 l/mn et on réalise par la suite un mûrissement de 30 mn à ce pH et à 95°C.

En fin de mûrissement, le pH est amené à 3,5 par ajout d'acide sulfurique. Ce pH est maintenu à 3,5 pendant 30 mn.

Après arrêt du chauffage, le mélange est filtré et le gâteau obtenu est lavé à l'eau désionisée jusqu'à obtention d'un filtrat de conductivité égale à 2000 µS/cm. Le gâteau obtenu après lavage est dispersé en présence d'eau désionisée pour former une suspension de concentration en silice égale à 10 %. Le pH de la suspension est ajusté à 6 par ajout d'acide acétique.

On procède à une deuxième filtration suivie d'un lavage à l'eau de manière à ajuster la conductivité à 500 µS/cm et d'un lavage avec de l'eau de pH ajusté à 5 par l'acide acétique de manière à ajuster le pH à 5,5.

On vérifie par la suite que la relation suivante est vérifiée :

$$pH \leq 8,20 - 0,91 \log (D)$$

Le gâteau est ensuite délité et la silice est séchée par atomisation. On procède enfin à un broyage de la silice obtenue sur un broyeur à couteaux pour obtenir une poudre dont le diamètre moyen des agglomérats mesuré au COUNTER-COULTER est de 8 µm.

Les caractérisations physico-chimiques de la silice ainsi obtenue sont regroupées dans le tableau ci-dessous :

| | |
|---|---|
| Surface BET $m^2/g$ | 65 |
| Surface CTAB $m^2/g$ | 60 |
| Prise DOP ml/100 g silice | 125 |
| Volume poreux Hg $cm^3/g$ | 1,90 |
| pH (5 % d'eau) | 6,2 |
| Indice de réfraction | 1,450 |
| Translucidité % | 90 |
| $SO_4^=$ ppm | 100 |
| $Na^+$ ppm | 60 |
| $Al^{3+}$ ppm | 200 |
| $Fe^{3+}$ ppm | 120 |
| $Ca^{2+}$ ppm | 30 |
| $Cl^-$ ppm | 20 |
| C ppm | 5 |

On récapitule dans le Tableau I les caractéristiques de chimie de surface de la silice de l'invention et l'on donne dans le Tableau I les résultats de la compatibilité avec les composés organiques aminés et dans le Tableau II les résultats de compatibilité avec les composants classiques des formulations de dentifrice : chlorhexidine, fluorure, zinc, pyrophosphate.

EXEMPLE 2

Dans un réacteur équipé d'un système de régulation de température et de pH et d'un système d'agitation à hélice (Mixel), on introduit 5,30 l de silicate de sodium de concentration en silice de 135 g/l et de rapport molaire $SiO_2/Na_2O$ = 3,5 et 15,00 l d'eau permutée de conductivité 1 µS/cm.

Après la mise en marche de l'agitation (350 t/mn), le pied de cuve ainsi constitué est chauffé à 90°C.

Lorsque la température est atteinte, on procède à l'addition d'acide sulfurique de concentration 80 g/l avec un débit constant de 0,38 l/mn pour amener le pH à 9,5.

On procède par la suite à l'addition simultanée de 44,70 l de silicate de sodium de concentration en silice de 135 g/l, de rapport molaire $SiO_2/Na_2O$ = 3,5 et de débit égal à 0,745 l/mn et de 25,30 l d'acide sulfurique à 80 g/l. Le débit d'acide sulfurique est ajusté de manière à maintenir le pH du milieu réactionnel à une valeur constante de 9,5.

Après 60 mn d'addition, on arrête l'addition de silicate de sodium et on continue l'ajout d'acide sulfurique avec un débit de 0,350 l/mn jusqu'à stabilisation du pH du mélange réactionnel à 8,0. Pendant cette phase, la température du milieu est portée à 95°C. On réalise, par la suite, un mûrissement de 30 mn à ce pH et à 95°C. Pendant le mûrissement, le pH est maintenu à 8 par ajout d'acide.

En fin de mûrissement, le pH est amené à 5,0 par ajout d'acide sulfurique avec un débit de 0,400 l/mn et on réalise par la suite un mûrissement de 30 mn à ce pH et à 95°C.

En fin de mûrissement, le pH est amené à 3,5 par ajout d'acide sulfurique. Ce pH est maintenu à 3,5 pendant 30 mn.

Après arrêt du chauffage, le mélange est filtré et le gâteau obtenu est lavé à l'eau désionisée jusqu'à obtention d'un filtrat de conductivité égale à 2000 μS/cm.

Le gâteau est ensuite délité en présence d'eau pour former une suspension à 20 % en silice et on ajuste le pH à 5,1 de manière à ce que la relation suivante soit vérifiée :

$$pH \leq 8,20 - 0,91 \log (D)$$

La silice est séchée par atomisation et broyée sur un broyeur à couteaux pour obtenir une poudre dont le diamètre moyen des agglomérats est de 8 μm.

Les caractérisations physico-chimiques de la silice ainsi obtenue sont regroupées dans le tableau ci-dessous :

| | |
|---|---|
| Surface BET $m^2/g$ | 100 |
| Surface CTAB $m^2/g$ | 80 |
| Prise DOP ml/100 g silice | 200 |
| Volume poreux Hg $cm^3/g$ | 3,35 |
| pH (5 % eau) | 6.5 |
| Indice de réfraction | 1,455 |
| Translucidité % | 95 |
| $SO_4^=$ % | 0,5 |
| $Na^+$ % | 0,25 |
| $Al^{3+}$ ppm | 350 |
| $Fe^{3+}$ ppm | 120 |
| $Ca^{2+}$ ppm | 50 |
| $Cl^-$ ppm | 20 |
| C ppm | 5 |

On récapitule dans le Tableau I les caractéristiques de chimie de surface de la silice de l'invention et l'on donne dans le Tableau I les résultats de la compatibilité avec les composés organiques aminés et dans le Tableau II les résultats de compatibilité avec les composants classiques des formulations de dentifrice : chlorhexidine, fluorure, zinc, pyrophosphate.

EXEMPLE 3

Dans un réacteur équipé d'un système de régulation de température et de pH et d'un système d'agitation à hélice (Mixel), on introduit 5,60 l de silicate de sodium de concentration en silice de 135 g/l et de rapport molaire $SiO_2/Na_2O$ = 3,5.

Après la mise en marche de l'agitation (350 t/mn), le pied de cuve ainsi constitué est chauffé à 85°C.

Lorsque la température est atteinte, on procède à l'addition d'acide sulfurique de concentration 85 g/l pré-chauffé à 70°C avec un débit constant de 0,50 l/mn pour amener le pH à 9,7.

On procède par la suite à l'addition simultanée de 52,64 l de silicate de sodium de concentration en silice de 135 g/l, de rapport molaire $SiO_2/Na_2O$ = 3,5 et de débit égal à 0,745 l/mn et de 30,00 l d'acide sulfurique à 85 g/l. Le débit d'acide sulfurique est ajusté de manière à maintenir le pH du milieu réactionnel à une valeur constante de 9,7. L'addition simultanée est effectuée à 85°C en utilisant des réactifs préchauffés à 70°C.

Après 45 mn d'addition, on arrête l'addition de silicate de sodium et on continue l'ajoute d'acide sulfurique avec un débit de 0,450 l/mn jusqu'à stabilisation du pH du mélange réactionnel à 8,0. Pendant cette phase, la température du

milieu est portée à 95°C. On réalise, par la suite, un mûrissement de 10 mn à ce pH et à 95°C. Pendant le mûrissement, le pH est maintenu à 8 par ajout d'acide.

En fin de mûrissement, le pH est amené à 5,0 par ajout d'acide sulfurique avec un débit de 0,750 l/mn et on réalise par la suite un mûrissement de 15 mn à ce pH et à 95°C.

En fin de mûrissement, le pH est amené à 3,7 par ajout d'acide sulfurique. Ce pH est maintenu à 3,7 pendant 60 mn.

Après arrêt du chauffage, le mélange est filtré et le gâteau obtenu est lave à l'eau désionisée jusqu'à obtention d'un filtrat de conductivité égale à 2500 µS/cm.

Le gâteau et ensuite délité en présence d'eau pour former une suspension à 20 % en silice et on ajuste le pH à 5,5 de manière à ce que la relation suivante soit vérifiée :

$$pH \leq 7,5 - 0,70 \log (D)$$

La silice est séchée par atomisation et broyée sur un broyeur à couteaux pour obtenir une poudre dont le diamètre moyen des agglomérats est de 8 µm.

Les caractérisations physico-chimiques de la silice ainsi obtenue sont regroupées dans le tableau ci-dessous :

| | |
|---|---|
| Surface BET $m^2/g$ | 200 |
| Surface CTAB $m^2/g$ | 55 |
| Prise DOP ml/100 g silice | 110 |
| Volume poreux Hg $cm^3/g$ | 2,65 |
| pH (5 % eau) | 7,0 |
| Indice de réfraction | 1,460 |
| Translucidité % | 85 |
| $SO_4^-$ % | 200 |
| $Na^+$ % | 60 |
| $Al^{3+}$ ppm | 150 |
| $Fe^{3+}$ ppm | 120 |
| $Ca^{2+}$ ppm | 50 |
| $Cl^-$ ppm | 20 |
| C ppm | 5 |

On récapitule dans le Tableau I, ci-après, les caractéristiques de chimie de surface des silices de l'invention décrites dans les exemples 1 à 3.

On donne également dans le Tableau I, les résultats de compatibilité des silices de l'invention avec les composés organiques aminés.

On mentionne dans le Tableau II, ci-après, les résultats de compatibilité avec les composants classiques intervenant dans les formulations de dentifrices : chlorhexidine, fluorure, zinc, pyrophosphate.

A titre comparatif, on consigne dans les Tableaux I et II, les caractéristiques et les différentes compatibilités de silices commerciales dont la liste ci-après définie constitue une gamme représentative des silices classiques.

| | |
|---|---|
| S81 | : Syloblanc 81 (GRACE) |
| Z113 | : Zeodent 113 (HUBER) |
| Sid12 | : Sident 12 (DEGUSSA) |
| Syl15 | : Sylox 15 (GRACE) |
| T73 | : Tixosil 73 (RHONE-POULENC) |
| T83 | : Tixosil 83 (RHONE-POULENC) |

Tableau I

| Caractéristiques physico-chimiques et compatibilité avec les composés organiques aminés des silices de l'invention et des silices classiques. | | | | | | | | |
|---|---|---|---|---|---|---|---|---|
| | Caractéristiques Physico-Chimiques des Silices | | | | | % compatibilité | | |
| silice | pH/log(C) | pH/log(D) | SE | Ho | PZC | AF | Béta | CHx |
| S81 | 4,7-0,75x | 7,0-0,62z | - 0,17 | ≤ 2 | 2,2 | 0 | 0 | 0 |
| Z113 | 8,1-0,94x | 10-1,0z | - 0,70 | ≤ 3 | 2,5 | 0 | 0 | 0 |
| Sid12 | 7,6-0,55x | 8,5-0,60z | - 0,20 | ≤ 3 | 2,8 | 0 | 0 | 0 |
| Syl15 | 8,1-0,70x | 9,2-0,74z | - 0,94 | ≤ 3 | 2,5 | 0 | 0 | 0 |
| T73 | 8,6-0,81x | 10-0,87z | - 0,20 | ≤ 3 | 3,0 | 0 | 0 | 0 |
| T83 | 7,5-0,60x | 8,6-0,60z | - 0,18 | ≤ 3 | 2,5 | 0 | 0 | 0 |
| Ex 1 | 7,5-0,30x | 8,0-0,50z | - 0,00 | ≥ 4 | 4,2 | 85 | 60 | 95 |
| Ex 2 | 6,5-0,80x | 8,2-0,90z | - 0,10 | ≥ 4 | 4,5 | 85 | 50 | 30 |
| Ex 3 | 7,0-0,40x | 7,4-0,60z | - 0,06 | ≥ 4 | 4,0 | 80 | 50 | 90 |

La signification des symboles utilisés dans le tableau ci-dessus est donnée ci-dessous :

- pH/log(C) représente l'équation pH = b-a.log(C) où a et b sont deux constantes et C est le pourcentage en poids de silice dans la suspension.
- pH/log(D) représente l'équation pH = b'-a'log(D) où b' et a' sont deux constantes et D est la conductivité de la suspension de silice en µSiemens/cm.
- SE représente l'effet de suspension mesuré par la relation SE = pH suspension - pH surnageant définie par ailleurs
- Ho est la constante de Hammett
- PZC représente le pH pour lequel la charge de surface de la silice est nulle.
- AF, AFc, Béta, aBéta et CHx représentent les pourcentages de Compatibilité en amines fluorées, en alkylbétaine et en chlorhexidine respectivement. Ces grandeurs sont définies par ailleurs.

Les pourcentages de compatibilité obtenus avec l'amine fluorée AFc et l'alkylbétaine aBéta sont similaires à ceux obtenus respectivement pour AF et Béta.

Tableau II

| Compatibilités des silices avec les molécules actives : | | | | | | |
|---|---|---|---|---|---|---|
| | % Compatibilité | | | | | |
| silice | $P2O7^{=}$ | $Zn^{++}$ | $F^{-}$ | AF | Béta | CHx |
| S81 | 80 | 0 | 90 | 0 | 0 | 0 |
| Z113 | 90 | 0 | 95 | 0 | 0 | 0 |
| Sid12 | 80 | 10 | 90 | 0 | 0 | 0 |
| Syl15 | 80 | 0 | 90 | 0 | 0 | 0 |
| T73 | 90 | 20 | 90 | 0 | 0 | 0 |
| T83 | 95 | 10 | 95 | 0 | 0 | 0 |
| Ex 1 | 95 | 80 | 95 | 85 | 60 | 95 |
| Ex 2 | 90 | 75 | 95 | 85 | 50 | 30 |
| Ex 3 | 95 | 80 | 95 | 80 | 50 | 90 |

Les résultats du Tableau I ci-dessus montrent que les silices de l'invention compatibles notamment avec les composés organiques aminés se différentient de façon nette des silices classiques par les relations suivantes :

$$pH \le 8{,}5\text{-}0{,}40 \log (D) \text{ et } pH \ge 7{,}0\text{-}0{,}60 \log (D)$$

$$pH \le 7{,}5\text{-}0{,}70 \log (C) \text{ et } pH \ge 5{,}0\text{-}0{,}50 \log (C)$$

**Revendications**

**Revendications pour les Etats contractants suivants : AT, BE, CH, DE, DK, FR, GB, IT, LI, LU, NL, SE**

**1.** Silice caractérisée par le fait qu'elle présente une compatibilité avec les composés organiques aminés d'au moins 30 % et qu'elle conduit à une suspension aqueuse dont le pH varie en fonction de sa concentration dans l'aire définie par les deux inéquations :

$$pH \le 7{,}5 - 0{,}7 \log (C) \qquad \text{(Ia)}$$

et

$$pH \ge 5{,}0 - 0{,}5 \log (C) \qquad \text{(Ib)}$$

et dont le pH varie en fonction de sa conductivité électrique dans l'aire définie par les deux inéquations :

$$pH \le 8{,}5 - 0{,}4 \log (D) \qquad \text{(IIa)}$$

et

$$pH \ge 7{,}0 - 0{,}6 \log (D) \qquad \text{(IIb)}$$

- dans les inéquations (Ia) et (Ib),
  . (C) représente la concentration pondérale de la suspension aqueuse de silice exprimée en % de $SiO_2$,
- dans les inéquations (IIa) et (IIb),
  . (D) représente la conductivité électrique de la suspension aqueuse de silice exprimée en microsie-

mens.$cm^{-1}$.

**2.** Silice selon la revendication 1 caractérisée par le fait qu'elle présente une chimie de surface telle que sa fonction d'acidité Ho soit d'au moins 4,0.

**3.** Silice selon l'une des revendications 1 et 2 caractérisée par le fait qu'elle présente une chimie de surface telle que le nombre de $OH^-$ exprimé en $OH^-/nm^2$ soit inférieur ou égal à 12, de préférence compris entre 6 et 10.

**4.** Silice selon l'une des revendications 1 à 3 caractérisée par le fait qu'elle présente un point de charge nulle (PZC) d'au moins 4, de préférence compris entre 4 et 6.

**5.** Silice selon l'une des revendications 1 à 4 caractérisée par le fait qu'elle présente une compatibilité avec les composés organiques aminés d'au moins 50 %, plus particulièrement d'au moins 80 %.

**6.** Silice selon l'une des revendications 1 à 5 caractérisée par le fait qu'elle présente une compatibilité avec les composés organiques aminés choisis dans le groupe formé par les amines fluorées, les oxydes d'amines, les alkylamines et les alkylbétaines.

**7.** Silice selon la revendication 6 caractérisée par le fait que composé organique aminé est l'hydrofluorure de cétylamine, le dihydrofluorure de bis-(hydroxyéthyl)aminopropyl N-hydroxyéthyl octadécylamine, un oxyde d'amine de formule

$$R(CH_3)_2\,N \rightarrow O,$$

une alkylbétaine de formule $R\text{-}N^+(CH_3)_2\text{-}CH_2\text{-}COO^-$, une alkylamidoalkylbétaine de formule $R\text{-}CO\text{-}NH_2\text{-}(CH_2)_3\text{-}N^+(CH_3)_2\text{-}CH_2\text{-}COO^-$ ; dans lesdites formules R représente un radical alkyle linéaire ou ramifié ayant de 10 à 24 atomes de carbone.

**8.** Silice selon la revendication 1 caractérisée par le fait qu'elle présente une compatibilité avec les cations métalliques bivalents et plus choisis dans les groupes 2a, 3a, 4a et 8 de la classification périodique d'au moins 50 %, plus particulièrement d'au moins 70 %.

**9.** Silice selon la revendication 8 caractérisée par le fait que le cation métallique est le calcium, le strontium, le baryum, l'aluminium, l'indium, le germanium, l'étain, le plomb, le manganèse, le fer, le nickel, le zinc, le titane, le zirconium, le palladium.

**10.** Silice selon l'une des revendications 8 et 9 caractérisée par le fait que la cation métallique est sous forme de sels minéraux, chlorure, fluorure, nitrate, phosphate, sulfate ou sous forme de sels organiques acétate, citrate.

**11.** Silice selon la revendication 10 caractérisée par le fait que le cation métallique est sous forme de citrate de zinc, de sulfate de zinc, de chlorure de strontium, de fluorure d'étain.

**12.** Silice selon la revendication 1 caractérisée par le fait qu'elle présente en outre une compatibilité avec les produits du type guanidine, notamment la chlorhexidine d'au moins de 30 % et plus particulièrement d'au moins 60 %.

**13.** Silice selon l'une des revendications 1 à 12 caractérisée par le fait qu'elle présente une teneur en anions du type $SO_4^{2-}$, $Cl^-$, $NO_3^-$, $PO_3^-$, $CO_3^{2-}$ d'au plus $5.10^{-3}$ moles pour 100 g de silice.

**14.** Silice selon la revendication 13 caractérisée par le fait qu'elle présente une teneur en anions précités d'au plus $1.10^{-3}$, plus particulièrement d'au plus $0{,}2.10^{-3}$ moles pour 100 g de silice.

**15.** Silice selon l'une des revendications 1 à 12 caractérisée par le fait qu'elle présente une teneur en sulfate exprimée en $SO_4^{2-}$ d'au plus 0,1 %, de préférence d'au plus 0,05 % et plus particulièrement d'au plus 0,01 %.

**16.** Silice selon l'une des revendications 1 à 15 caractérisée par le fait qu'elle présente une teneur en cations métalliques bivalents et plus d'au plus 1000 ppm.

**17.** Silice selon la revendications 16 caractérisée par le fait que la teneur en aluminium est d'au plus 500 ppm, la teneur en fer d'au plus 200 ppm, la teneur en calcium d'au plus 500 ppm et, plus particulièrement, d'au plus 300 ppm.

**18.** Silice selon l'une des revendications 1 à 17 caractérisée par le fait qu'elle présente une teneur en carbone d'au plus 50 ppm et plus particulièrement d'au plus 10 ppm.

**19.** Silice selon l'une des revendications 1 à 18 caractérisée par le fait qu'elle présente un pH d'au plus 8 et plus particulièrement compris entre 6,0 et 7,5.

**20.** Silice selon l'une des revendications 1 à 19 caractérisée par le fait qu'elle présente une surface BET comprise entre 40 et 600 $m^2/g$.

**21.** Silice selon l'une des revendications 1 à 20 caractérisée par le fait qu'elle présente une surface CTAB comprise entre 40 et 400 $m^2/g$.

**22.** Silice selon l'une des revendications 1 à 21 du type abrasif caractérisée par le fait qu'elle présente une surface BET comprise entre 40 et 300 $m^2/g$.

**23.** Silice selon la revendication 22 caractérisée par le fait qu'elle présente une surface CTAB comprise entre 40 et 100 $m^2/g$.

**24.** Silice selon l'une quelconque des revendications 1 à 21 du type épaississant caractérisée par le fait qu'elle présente une surface BET comprise entre 120 et 450 $m^2/g$, plus particulièrement 120 et 200 $m^2/g$.

**25.** Silice selon la revendication 24, caractérisée par le fait qu'elle présente une surface CTAB comprise entre 120 et 400 $m^2/g$.

**26.** Silice selon l'une des revendications 1 à 21 du type bifonctionnel, caractérisée par le fait qu'elle présente une surface BET comprise entre 80 et 200 $m^2/g$.

**27.** Silice selon la revendication 26 caractérisée par le fait qu'elle présente une surface CTAB comprise entre 80 et 200 $m^2/g$.

**28.** Silice selon l'une des revendications 1 à 27 caractérisée par le fait qu'elle présente une prise d'huile comprise entre 80 et 500 $cm^3/100$ g.

**29.** Silice selon l'une des revendications 1 à 28 caractérisée par le fait qu'elle présente une taille moyenne de particules comprise entre 1 et 10 µm.

**30.** Silice selon l'une des revendications 1 à 29 caractérisée par le fait qu'elle présente une densité apparente comprise entre 0,01 et 0,3.

**31.** Silice selon l'une des revendications 1 à 30 caractérisée par le fait qu'il s'agit d'une silice de précipitation.

**32.** Procédé de préparation d'une silice décrite dans l'une des revendications 1 à 31 caractérisé par le fait qu'il consiste à faire réagir un silicate avec un acide conduisant ainsi à une suspension ou un gel de silice, à effectuer un premier mûrissement à un pH supérieur ou égal à 6 et inférieur ou égal à 8,5, puis un deuxième mûrissement à un pH inférieur ou égal à 6,0 à effectuer un troisième mûrissement à un pH inférieur ou égal à 5,0; à séparer la silice, à la soumettre à un lavage à l'eau jusqu'à ce qu'il conduise à une suspension aqueuse dont le pH mesuré sur une suspension à 20 % de $SiO_2$ réponde à l'équation suivante :

$$pH = d - e \log (D) \qquad (III)$$

dans l'équation (III)

. e est une constante supérieure ou égale à 0,6 et inférieure ou égale à 1,0,
. d est une constante supérieure ou égale à 7,0 et inférieure ou égale à 8,5,
. (D) représente la conductivité électrique de la suspension aqueuse de silice exprimée en microsiemens.$cm^{-1}$ ;
enfin à la sécher.

**33.** Procédé selon la revendication 32 caractérisé par le fait qu'il consiste à préparer la suspension ou le gel de silice en ajoutant simultanément le silicate et l'acide sur un pied de cuve qui peut être de l'eau, une dispersion colloïdale

de silice contenant de 0 à 150 g/l de silice exprimée en $SiO_2$, un silicate ou un sel minéral ou organique, de préférence de métal alcalin.

34. Procédé selon la revendication 33 caractérisé par le fait que l'addition des deux réactifs se fait de manière simultanée de telle sorte que le pH soit maintenu constant entre 4 et 10, de préférence entre 8,5 et 9,5.

35. Procédé selon l'une des revendications 33 et 34 caractérisé par le fait que la température est comprise entre 60°C et 95°C.

36. Procédé selon la revendication 33 caractérisé par le fait que l'on prépare la dispersion colloïdale de silice contenant de 20 à 150 g/l de silice en chauffant une solution aqueuse de silicate entre 60°C et 95°C et à ajouter l'acide dans ladite solution aqueuse jusqu'à obtention d'un pH compris entre 8,0 et 10,0, de préférence, égal à 9,5.

37. Procédé selon la revendication 32 caractérisé par le fait qu'il consiste à préparer une suspension ou un gel de silice en ajoutant progressivement l'acide dans un pied de cuve contenant le silicate jusqu'à obtention du pH de mûrissement désiré à une température comprise, entre 60°C et 95°C.

38. Procédé selon l'une des revendications 32 à 35 caractérisé par le fait que l'on effectue un premier mûrissement de la suspension ou du gel de silice à un pH compris entre 6 et 8,5, de préférence égal à 8,0 à une température comprise entre 60°C et 100°C, de préférence égale à 95°C.

39. Procédé selon l'une des revendications 32 à 38 caractérisé par le fait que l'on effectue un deuxième mûrissement de la suspension ou du gel de silice à un pH compris entre 5 et 6, de préférence égal à 5,5, à une température comprise entre 60°C et 100°C, de préférence égale à 95°C.

40. Procédé selon l'une des revendications 32 à 39 caractérisé par le fait que l'on effectue un troisième mûrissement de la suspension ou du gel de silice à un pH compris entre 3 et 5, de préférence égal à 4, à une température comprise entre 60°C et 100°C, de préférence égale à 95°C.

41. Procédé selon la revendication 32 caractérisé par le fait que l'on effectue un lavage à l'eau ou à l'aide d'une solution acide.

42. Procédé selon la revendication 32 caractérisé par le fait que l'on effectue un lavage effectué jusqu'à ce que la conductivité du filtrat de lavage soit d'au plus 200 microsiemens.$cm^{-1}$.

43. Procédé selon la revendication 42 caractérisé par le fait que l'on effectue un lavage à l'eau ou à l'aide d'une solution acide.

44. Procédé selon l'une des revendications 41 et 43 caractérisé par le fait que la solution acide précitée est une solution d'un acide organique notamment d'un acide complexant.

45. Procédé selon la revendication 44 caractérisé par le fait que l'acide organique précité est choisi dans le groupe des acides carboxyliques, des acides dicarboxyliques, des acides aminocarboxyliques, des acides hydrocarboxyliques.

46. Procédé selon l'une des revendications 44 et 45 caractérisé par le fait que l'acide organique est choisi dans le groupe comprenant les acides acétique, gluconique, tartrique, citrique, maléique et glycérique.

47. Composition dentifrice caractérisée par le fait qu'elle contient une silice selon l'une des revendications 1 à 31 ou une silice préparée par le procédé selon l'une des revendications 32 à 46.

48. Composition dentifrice selon la revendication 47 caractérisée par le fait qu'elle comprend au moins un élément choisi dans le groupe comprenant le fluor, les phosphates, les guanidines.

49. Composition dentifrice selon la revendication 47 caractérisée par le fait qu'elle comprend au moins un élément choisi dans le groupe comprenant les composés organiques aminés, les cations bivalents et plus.

50. Composition dentifrice selon la revendication 47 caractérisée par le fait qu'elle comprend au moins un composé organique aminé choisi dans le groupe formé par les amines fluorées, les oxydes d'amines, les alkylamines et les alkylbétaines.

**51.** Composition dentifrice selon la revendication 50 caractérisée par le fait que le composé organique aminé est l'hydrofluorure de cétylamine, le dihydrofluorure de bis-(hydroxyéthyl)aminopropyl N-hydroxyéthyl octadécylamine, un oxyde d'amine de formule

$$R(CH_3)_2\ N \rightarrow O,$$

une alkylbétaine de formule $R\text{-}N^+\ (CH_3)_2\text{-}CH_2\text{-}COO^-$, une alkylamidoalkylbétaine de formule $R\text{-}CO\text{-}NH_2\text{-}(CH_2)_3\text{-}N^+(CH_3)_2\text{-}CH_2\text{-}COO^-$ , dans lesdites formules R représente un radical alkyle linéaire ou ramifié ayant de 10 à 24 atomes de carbone.

**52.** Composition dentifrice selon la revendication 49 caractérisée par le fait qu'elle comprend au moins un cation métallique bivalent et plus choisi dans le groupe 2a, 3a, 4a et 8 de la classification périodique.

**53.** Composition dentifrice selon la revendication 52 caractérisée par le fait que le cation métallique est le calcium, le strontium, le baryum, l'aluminium, l'indium, le germanium, l'étain, le plomb, le manganèse, le fer, le nickel, le zinc, le titane,le zirconnium, le palladium.

**54.** Composition dentifrice selon l'une des revendications 52 et 53 caractérisée par le fait que le cation métallique est sous forme de sels minéraux, chlorure, fluorure, nitrate, phosphate, sulfate ou sous forme de sels organiques acétate, citrate.

**55.** Composition dentifrice selon l'une des revendications 52 à 54 caractérisée par le fait que le cation métallique est sous forme de citrate de zinc, de sulfate de zinc, de chlorure de strontium, de fluorure d'étain.

**56.** Composition dentifrice selon la revendication 47 caractérisée par le fait qu'elle comprend de la chlorhexidine.

**Revendications pour l'Etat contractant suivant : ES**

**1.** Procédé de préparation d'une silice, cette silice présentant une compatibilité avec les composés organiques aminés d'au moins 30 % et conduisant à un suspension aqueuse dont le pH varie en fonction de sa concentration dans l'aire définie par les deux inéquations:

$$pH \leq 7{,}5 - 0{,}7 \log (C) \qquad (Ia)$$

et

$$pH \geq 5{,}0 - 0{,}5 \log (C) \qquad (Ib)$$

et dont le pH varie en fonction de sa condustivité électrique dans l'aire définie par les deux inéquations:

$$pH \leq 8{,}5 - 0{,}4 \log (D) \qquad (IIa)$$

et

$$pH \geq 7{,}0 - 0{,}6 \log (D) \qquad (IIb)$$

- dans les inéquations (Ia) et (Ib),

  . (C) représente la concentration pondérale de la suspension aqueuse de silice exprimée en % de $SiO_2$,

- dans les inéquations (IIa) et (IIb),

  . (D) représente la conductivité électrique de la suspension aqueuse de silice exprimée en microsiemens.$cm^{-1}$,

caractérisé par le fait qu"il consiste à faire réagir un silicate avec un acide consuisant ainsi à une suspension ou un gel de silice, à effectuer un premier mûrissement à un pH supérieur ou égal à 6 et inférieur ou égal à 8,5, puis un deuxième mûrissement à un pH inférieùr ou égal à 6,0; à effectuer un troisième mûrissement à un pH inférieur ou égal à 5,0; à séparer la silice, à la soumettre à un lavage à l'eau jusqu'à ce qu'il conduise à une suspension

aqueuse dont le pH mesuré sur une suspension à 20 % de $SiO_2$ réponde à l'équation suivante:

$$pH = d - e \log (D) \qquad (III)$$

dans l'équation (III)

- . e est une constante supérieure ou égale à 0,6 et inférieure ou égale à 1,0,
- . d est une constante supérieure ou égale à 7,0 et inférieure ou égale à 8,5,
- . (D) représente la conductivité électrique de la suspension aqueuse de silice exprimée en microsiemens.$cm^{-1}$; enfin à la sècher.

**2.** Procédé selon la revendication 1 caractérisé par le fait que la silice présente une chimie de surface telle que sa fonction d'acidité Ho soit d'au moins 4,0.

**3.** Procédé selon l'une des revendications 1 et 2 caractérisé par le fart que la silice présente une chimie de surface telle que le nombre de $OH^-$ exprimé en $OH^-/nm^2$ soit inférieur ou égal à 12, de préférence compris entre 6 et 10.

**4.** Procédé selon l'une des revendications 1 à 3 caractérisé par le fait que la silice présente un point de charge nulle (PZC) d'au moins 4, de préférence compris entre 4 et 6.

**5.** Procédé selon l'une des revendications 1 à 4 caractérisée par le fait que la silice présente une compatibilité avec les composés organiques aminés d'au moins 50 %, plus particulièrement d'au moins 80 %.

**6.** Procédé selon l'une des revendications 1 à 5 caractérisé par le fait que la silice présente une compatibilité avec les composés organiques aminés choisis dans le groupe formé par les amines fluorées, les oxydes d'amines, les alkylamines et les alkylbétaines.

**7.** Procédé selon la revendication 6 caractérisé par le fait que le composé organique aminé est l'hydrofluorure de cétylamine, le dihydrofluorure de bis-(hydroxyéthyl)aminopropyl N-hydroxyéthyl octadécylamine, un oxyde d'amine de formule

$$R(CH_3)_2 N \rightarrow\!> O,$$

une alkylbétaine de formule $R-N^+(CH_3)_2-CH_2-COO^-$, une alkylamidoalkylbétaine de formule $R-CO-NH_2-(CH_2)_3-N^+(CH_3)_2-CH_2-COO^-$ dans lesdites formules R représentant un radical alkyle linéaire ou ramifié ayant de 10 à 24 atomes de carbone.

**8.** Procédé selon la revendication 1 caractérisé par le fait que la silice présente une compatibilité avec les cations métalliques bivalents et plus choisis dans les groupes 2a, 3a, 4a et 8 de la classification périodique d'au moins 50 %, plus particulièrement d'au moins 70 %.

**9.** Procédé selon la revendication 8 caractérisé par le fait que le cation métallique est le calcium, le strontium, le baryum, l'aluminium, l'indium, le germanium, l'étain, le plomb, le manganèse, le fer, le nickel, le zinc, le titane, le zirconium, le palladium.

**10.** Procédé selon l'une des revendications 8 et 9 caractérisé par le fait que la cation métallique est sous forme de sels minéraux, chlorure, fluorure, nitrate, phosphate, sulfate ou sous forme de sels organiques acétate, citrate.

**11.** Procédé selon la revendication 10 caractérisé par le fait que le cation métallique est sous forme de citrate de zinc, de sulfate de zinc, de chlorure de strontium, de fluorure d'étain.

**12.** Procédé selon la revendication 1 caractérisé par le fait que la silice présente en outre une compatibilité avec les produits du type guanidine, notamment la chlorhexidine d'au moins de 30 % et plus particulièrement d'au moins 60 %.

**13.** Procédé selon l'une des revendications 1 à 12 caractérisé par le fait que la silice présente une teneur en anions du type $SO_4^{2-}$, $Cl^-$, $NO_3^-$, $PO_3^-$, $CO_3^{2-}$ d'au plus $5.10^{-3}$ moles pour 100 g de silice.

**14.** Procédé selon la revendication 13 caractérisé par le fait que la silice présente une teneur en anions précités d'au

plus $1.10^{-3}$, plus particulièrement d'au plus $0,2.10^{-3}$ moles pour 100 g de silice.

**15.** Procédé selon l'une des revendications 1 à 12 caractérisé par le fait que la silice présente une teneur en sulfate exprimée en $SO_4^{2-}$ d'au plus 0,1 %, de préférence d'au plus 0,05 % et plus particulièrement d'au plus 0,01 %.

**16.** Procédé selon l'une des revendications 1 à 15 caractérisé par le fait que la silice présente une teneur en cations métalliques bivalents et plus d'au plus 1000 ppm.

**17.** Procédé selon la revendication 16 caractérisé par le fait que la teneur en aluminium est d'au plus 500 ppm, la teneur en fer d'au plus 200 ppm, la teneur en calcium d'au plus 500 ppm et, plus particulièrement, d'au plus 300 ppm.

**18.** Procédé selon l'une des revendications 1 à 17 caractérisé par le fait que la silice présente une teneur en carbone d'au plus 50 ppm et plus particulièrement d'au plus 10 ppm.

**19.** Procédé selon l'une des revendications 1 à 18 caractérisé par le fait que la silice présente un pH d'au plus 8 et plus particulièrement compris entre 6,0 et 7,5.

**20.** Procédé selon l'une des revendications 1 à 19 caractérisé par le fait que la si ce présente une surface BET comprise entre 40 et 600 $m^2/g$.

**21.** Procédé selon l'une des revendications 1 à 20 caractérisé par le fait que la silice présente une surface CTAB comprise entre 40 et 400 $m^2/g$.

**22.** Procédé selon l'une des revendications 1 à 21 caractérisé par le fait que la silice est du type abrasif et présente une surface BET comprise entre 40 et 300 $m^2/g$.

**23.** Procédé selon la revendication 22 caractérisé par le fait que la silice présente une surface CTAB comprise entre 40 et 100 $m^2/g$.

**24.** Procédé selon l'une quelconque des revendications 1 à 21 caractérisé par le fait que la silice est du type épaississant et présente une surface BET comprise entre 120 et 450 $m^2/g$, plus particulièrement 120 et 200 $m^2/g$.

**25.** Procédé selon la revendication 24, caractérisé par le fait que la silice présente une surface CTAB comprise entre 120 et 400 $m^2/g$.

**26.** Procédé selon l'une des revendications 1 à 21 caractérisé par le fait que la silice est du type bifonctionnel et présente une surface BET comprise entre 80 et 200 $m^2/g$.

**27.** Procédé selon la revendication 26 caractérisé par le fait que la silice présente une surface CTAB comprise entre 80 et 200 $m^2/g$.

**28.** Procédé selon l'une des revendications 1 à 27 caractérisé par le fait que la silice présente une prise d'huile comprise entre 80 et 500 $cm^3/100$ g.

**29.** Procédé selon l'une des revendications 1 à 28 caractérisé par le fait que la silice présente une taille moyenne de particules comprise entre 1 et 10 $\mu$m.

**30.** Procédé selon l'une des revendications 1 à 29 caractérisé par le fait que la silice présente une densité apparente comprise entre 0,01 et 0,3.

**31.** Procédé selon l'une des revendications 1 à 30 caractérisé par le fait que la silice est une silice de précipitation.

**32.** Procédé selon l'une des revendications 1 à 31 caractérisé par le fait qu'il consiste à préparer la suspension ou le gel de silice en ajoutant simultanément le silicate et l'acide sur un pied de cuve qui peut être de l'eau, une dispersion colloïdale de silice contenant de 0 à 150 g/l de silice exprimée en $SiO_2$, un silicate ou un sel minéral ou organique, de préférence de métal alcalin.

**33.** Procédé selon la revendication 32 caractérisé par le fait que l'addition des deux réactifs se fait de manière simultanée de telle sorte que le pH soit maintenu constant entre 4 et 10, de préférence entre 8,5 et 9,5.

**34.** Procédé selon l'une des revendications 32 et 33 caractérisé par le fait que la température est comprise entre 60°C et 95°C.

**35.** Procédé selon la revendication 32 caractérisé par le fait que l'on prépare la dispersion colloïdale de silice contenant de 20 à 150 g/l de silice en chauffant une solution aqueuse de silicate entre 60°C et 95°C et à ajouter l'acide dans ladite solution aqueuse jusqu'à obtention d'un pH compris entre 8,0 et 10,0, de préférence, égal à 9,5.

**36.** Procédé selon l'une des revendications 1 à 31 caractérisé par le fait qu'il consiste à préparer une suspension ou un gel de silice en ajoutant progressivement l'acide dans un pied de cuve contenant le silicate jusqu'à obtention du pH de mûrissement désiré à une température comprise entre 60°C et 95°C.

**37.** Procédé selon l'une des revendications 1 à 34 caractérisé par le fait que l'on effectue un premier mûrissement de la suspension ou du gel de silice à un pH compris entre 6 et 8,5, de préférence égal à 8,0 à une température comprise entre 60°C et 100°C, de préférence égale à 95°C.

**38.** Procédé selon l'une des revendications 1 à 37 caractérisé par le fait que l'on effectue un deuxième mûrissement de la suspension ou du gel de silice à un pH compris entre 5 et 6, de préférence égal à 5,5, à une température comprise entre 60°C et 100°C, de préférence égale à 95°C.

**39.** Procédé selon l'une des revendications 1 à 38 caractérisé par le fait que l'on effectue un troisième mûrissement de la suspension ou du gel de silice à un pH compris entre 3 et 5, de préférence égal à 4, à une température comprise entre 60°C et 100°C, de préférence égale à 95°C.

**40.** Procédé selon l'une des revendications 1 à 31 caractérisé par le fait que l'on effectue un lavage à l'eau ou à l'aide d'une solution acide.

**41.** Procédé selon l'une des revendications 1 à 31 caractérisé par le fait que l'on effectue un lavage effectué jusqu'à ce que la conductivité du filtrat de lavage soit d'au plus 200 microsiemens.$cm^{-1}$.

**42.** Procédé selon la revendication 41 caractérisé par le fait que l'on effectue un lavage à l'eau ou à l'aide d'une solution acide.

**43.** Procédé selon l'une des revendications 40 et 42 caractérisé par le fait que la solution acide précitée est une solution d'un acide organique notamment d'un acide complexant.

**44.** Procédé selon la revendication 43 caractérisé par le fait que l'acide organique précité est choisi dans le groupe des acides carboxyliques, des acides dicarboxyliques, des acides aminocarboxyliques, des acides hydrocarboxyliques.

**45.** Procédé selon l'une des revendications 43 et 44 caractérisé par le fait que l'acide organique est choisi dans le groupe comprenant les acides acétique, gluconique, tartrique, citrique, maléique et glycérique.

**46.** Composition dentifrice caractérisée par le fait qu'elle contient une silice préparée par le procédé selon l'une des revendications 1 à 45.

**47.** Composition dentifrice selon la revendication 46 caractérisée par le fait qu'elle comprend au moins un élément choisi dans le groupe comprenant le fluor, les phosphates, les guanidines.

**48.** Composition dentifrice selon la revendication 46 caractérisée par le fait qu'elle comprend au moins un élément choisi dans le groupe comprenant les composés organiques aminés, les cations bivalents et plus.

**49.** Composition dentifrice selon la revendication 46 caractérisée par le fait qu'elle comprend au moins un composé organique aminé choisi dans le groupe formé par les amines fluorées, les oxydes d'amines, les alkylamines et les alkylbétaines.

**50.** Composition dentifrice selon la revendication 49 caractérisée par le fait que le composé organique aminé est l'hydrofluorure de cétylamine, le dihydrofluorure de bis-(hydroxyéthyl)aminopropyl N-hydroxyéthyl octadécylamine, un oxyde d'amine de formule

$$R(CH_3)_2\ N{\rightarrow}{>}O,$$

une alkylbétaine de formule $R\text{-}N^+(CH_3)_2\text{-}CH_2\text{-}COO^-$, une alkylamidoalkylbétaine de formule $R\text{-}CO\text{-}NH_2\text{-}(CH_2)_3\text{-}N^+(CH_3)_2\text{-}CH_2\text{-}COO^-$, dans lesdites formules R représentant un radical alkyle linéaire ou ramifié ayant de 10 à 24 atomes de carbone.

**51.** Composition dentifrice selon la revendication 46 caractérisée par le fait qu'elle comprend au moins un cation métallique bivalent et plus choisi dans le groupe 2a, 3a, 4a et 8 de la classification périodique.

**52.** Composition dentifrice selon la revendication 51 caractérisée par le fait que le cation métallique est le calcium, le strontium, le baryum, l'aluminium, l'indium, le germanium, l'étain, le plomb, le manganèse, le fer, le nickel, le zinc, le titane,le zirconium, le palladium.

**53.** Composition dentifrice selon l'une des revendications 51 et 52 caractérisée par le fait que le cation métallique est sous forme de sels minéraux, chlorure, fluorure, nitrate, phosphate, sulfate ou sous forme de sels organiques acétate, citrate.

**54.** Composition dentifrice selon l'une des revendications 51 à 53 caractérisée par le fait que le cation métallique est sous forme de citrate de zinc, de sulfate de zinc, de chlorure de strontium, de fluorure d'étain.

**55.** Composition dentifrice selon la revendication 46 caractérisée par le fait qu'elle comprend de la chlorhexidine.

**Claims**

**Claims for the following Contracting States : AT, BE, CH, DE, DK, FR, GB, IT, LI, LU, NL, SE**

**1.** Silica, characterised in that it has a compatibility with organic amino compounds of at least 30%, and that it leads to an aqueous suspension whose pH varies as a function of its concentration in the area defined by the two inequations:

$$Ph \leq 7.5\text{-}0.7 \log (C) \qquad (Ia)$$

and

$$pH \geq 5.0\text{-}0.5 \log (C) \qquad (Ib)$$

and whose pH varies as a function of its electrical conductivity in the area defined by the two inequations:

$$pH \leq 8.5\text{-}0.4 \log (D) \qquad (IIa)$$

and

$$pH \geq 7.0\text{-}0.6 \log (D) \qquad (IIb)$$

- in the inequations (Ia) and (Ib),
  . (C) represents the weight concentration of the aqueous silica suspension expressed in % $SiO_2$,
- in the inequations (IIa) and (IIb),
  . (D) represents the electrical conductivity of the aqueous silica suspension expressed in microsiemens.$cm^{-1}$.

**2.** Silica according to Claim 1, characterised in that it has a surface chemistry such that its acidity function Ho is at least 4.0.

**3.** Silica according to either Claim 1 or Claim 2, characterised in that it has a surface chemistry such that the number of $OH^-$ expressed as $OH^-/nm^2$ is equal to or below 12 and preferably between 6 and 10.

4. Silica according to one of Claims 1 to 3, characterised in that it has a zero point charge (ZPC) of at least 4, preferably of between 4 and 6.

5. Silica according to one of Claims 1 to 4, characterised in that it has a compatibility with the organic amino compounds of at least 50%, more particularly of at least 80%.

6. Silica according to one of Claims 1 to 5, characterized in that it has a compatibility with the organic amino compounds selected from the group formed by fluorine-containing amines, amine oxides, alkyl amines and alkyl betaines.

7. Silica according to Claim 6, characterised in that the organic amino compound is cetyl amine hydrofluoride, bis-(hydroxyethyl)-aminopropyl-N-hydroxyethyl-octadecyl amine dihydrofluoride, an amine oxide of formula

$$R(CH_3)_2\ N \rightarrow 0,$$

an alkyl betaine of formula $R\text{-}N^+\ (CH_3)_2\text{-}CH_2\text{-}COO^-$, an alkyl amidoalkyl betaine of formula $R\text{-}CO\text{-}NH_2\text{-}(CH_2)_3\text{-}N^+(CH_3)_2\text{-}CH_2\text{-}COO^-$; in which formulae R represents a straight or branched-chain alkyl radical with 10 to 24 carbon atoms.

8. Silica according to Claim 1, characterised in that it has a compatibility with the divalent and higher valency metal cations selected from groups 2a, 3a, 4a and 8 of the periodic classification of at least 50%, more particularly of at least 70%.

9. Silica according to Claim 8, characterised in that the metal cation is calcium, strontium, barium, aluminium, indium, germanium, tin, lead, manganese, iron, nickel, zinc, titanium, zirconium, palladium.

10. Silica according to either Claim 8 or Claim 9, characterised in that the metal cation is in the form of the mineral salts, chloride, fluoride, nitrate, phosphate, sulphate, or in the form of the organic salts, acetate and citrate.

11. Silica according to Claim 10, characterised in that the metal cation is in the form of zinc citrate, zinc sulphate, strontium chloride or tin fluoride.

12. Silica according to Claim 1, characterised in that it also has a compatibility with products of the guanidine type, particularly chlorhexidine, of at least 30%, and, more particularly, of at least 60%.

13. Silica according to one of Claims 1 to 12, characterised in that it has a content of anions of type $SO_4^{2-}$, $Cl^-$, $NO_3^-$, $PO_3^-$, $CO_3^{2-}$ of at the most $5.10^{-3}$ moles/100 g of silica.

14. Silica according to Claim 13, characterised in that it has a content of the aforementioned anions of at the most $1.10^{-3}$, more particularly of at the most $0.2.10^{-3}$ moles/100 g of silica.

15. Silica according to one of Claims 1 to 12, characterised in that it has a sulphate content, expressed as $SO_4^{2-}$, of at the most 0.1%, preferably at the most 0.05%, and more particularly at the most 0.01%.

16. Silica according to one of Claims 1 to 15, characterised in that it has a content of divalent and higher valency metal cations of at the most 1000 ppm.

17. Silica according to Claim 16, characterised in that the aluminium content is at the most 500 ppm, the iron content is at the most 200 ppm, the calcium content is at the most 500 ppm, and, more particularly, at the most 300 ppm.

18. Silica according to one of Claims 1 to 17, characterised in that it has a carbon content of at the most 50 ppm, and, more particularly, of at the most 10 ppm.

19. Silica according to one of Claims 1 to 18, characterised in that it has a pH of at the most 8, and, more particularly, of between 6.0 and 7.5.

20. Silica according to one of Claims 1 to 19, characterised in that it has a BET surface of between 40 and 600 $m^2/g$.

21. Silica according to one of Claims 1 to 20, characterised in that it has a CTAB surface of between 40 and 400 $m^2/g$.

**22.** Silica according to one of Claims 1 to 21 of the abrasive type, characterised in that it has a BET surface of between 40 and 300 $m^2/g$.

**23.** Silica according to Claim 22, characterised in that it has a CTAB surface of between 40 and 100 $m^2/g$.

**24.** Silica according to any one of Claims 1 to 21 of the thickening type, characterised in that it has a BET surface of between 120 and 450 $m^2/g$, more particularly of between 120 and 200 $m^2/g$.

**25.** Silica according to Claim 24, characterised in that it has a CTAB surface of between 120 and 400 $m^2/g$.

**26.** Silica according to one of Claims 1 to 21 of the bifunctional type, characterised in that it has a BET surface of between 80 and 200 $m^2/g$.

**27.** Silica according to Claim 26, characterised in that it has a CTAB surface of between 80 and 200 $m^2/g$.

**28.** Silica according to one of Claims 1 to 27, characterised in that it has an oil absorption of between 80 and 500 $cm^3/100$ g.

**29.** Silica according to one of Claims 1 to 28, characterised in that it has an average particle size of between 1 and 10µm.

**30.** Silica according to one of Claims 1 to 29, characterised in that it has an apparent density of between 0.01 and 0.3.

**31.** Silica according to one of Claims 1 to 30, characterised in that it is a precipitation silica.

**32.** A process for the preparation of a silica described in one of Claims 1 to 31, characterised in that it consists in reacting a silicate with an acid, thus leading to a suspension or a silica gel, carrying out a first ageing operation at a pH equal to or higher than 6 and equal to or lower than 8.5, then a second ageing operation at a pH equal to or lower than 6.0; in carrying out a third ageing operation at a pH equal to or lower than 5.0; separating the silica, washing it in water until it leads to an aqueous suspension whereof the pH measured on a suspension with 20% $SiO_2$ corresponds to the following equation:

$$pH = d - e \log (D) \qquad (III)$$

in which equation (III)

. e is a constant equal to or higher than 0.6, and equal to or lower than 1.0,
. d is a constant equal to or higher than 7.0, and equal to or less than 8.5,
. (D) represents the electrical conductivity of the aqueous silica suspension expressed in $microsiemens.cm^{-1}$;
. finally, in drying it.

**33.** A process according to Claim 32, characterised in that it consists in preparing the suspension or silica gel by simultaneously adding the silicate and the acid to a sediment which can be water, a colloidal silica dispersion containing between 0 and 150 g/l silica expressed as $SiO_2$, a silicate or a mineral or organic salt, preferably an alkali metal salt.

**34.** A process according to Claim 33, characterised in that the two reagents are added simultaneously in such a way that the pH is kept constant at between 4 and 10, preferably at between 8.5 and 9.5.

**35.** A process according to either Claim 33 or Claim 34, characterised in that the temperature is between 60°C and 95°C.

**36.** A process according to Claim 33, characterised in that the colloidal silica dispersion containing between 20 and 150 g/l of silica is prepared by heating an aqueous silicate solution at between 60 and 95°C, and by adding acid to said aqueous solution until a pH is obtained of between 8.0 and 10.0, preferably of 9.5.

**37.** A process according to Claim 32, characterised in that it consists in preparing a suspension or a silica gel by gradually adding acid to a sediment containing silicate until the desired ageing pH is obtained at a temperature of between 60°C and 95°C.

**38.** A process according to one of Claims 32 to 35, characterised in that a first ageing operation is carried out on the suspension or silica gel at a pH of between 6 and 8.5, preferably of 8.0, at a temperature of between 60°C and 100°C, preferably of 95°C.

**39.** A process according to one of Claims 32 to 38, characterised in that a second ageing operation is carried out on the suspension or silica gel at a pH of between 5 and 6, preferably of 5.5, at a temperature of between 60°C and 100°C, preferably of 95°C.

**40.** A process according to one of Claims 32 to 39, characterised in that a third ageing operation is carried out on the suspension or silica gel, at a pH of between 3 and 5, preferably of 4, at a temperature of between 60°C and 100°C, preferably of 95°C.

**41.** A process according to Claim 32, characterised in that washing is carried out with water or with the aid of an acid solution.

**42.** A process according to Claim 32, characterised in that washing is carried out until the conductivity of the washing filtrate is at the most 200 microsiemens.$cm^{-1}$.

**43.** A process according to Claim 42, characterised in that washing takes place with water or with the aid of an acid solution.

**44.** A process according to either Claim 41 or Claim 43, characterised in that the aforementioned acid solution is a solution of an organic acid, in particular of a complexing acid.

**45.** A process according to Claim 44, characterised in that the afore-mentioned organic acid is selected from the group of carboxylic acids, dicarboxylic acids, aminocarboxylic acids, or hydrocarboxylic acids.

**46.** A process according to either Claim 44 or Claim 45, characterised in that the organic acid is selected from the group comprising acetic, gluconic, tartaric, citric, maleic and glyceric acids.

**47.** A dentifrice composition, characterised in that it contains a silica according to one of Claims 1 to 31, or a silica prepared using the process according to one of Claims 32 to 46.

**48.** A dentifrice composition according to Claim 47, characterised in that it comprises at least one element selected from the group comprising fluorine, phosphates, guanidines.

**49.** A dentifrice composition according to Claim 47, characterised in that it comprises at least one element selected from the group comprising organic amino compounds and divalent and higher valency cations.

**50.** A dentifrice composition according to Claim 47, characterised in that it comprises at least one organic amino compound selected from the group formed by fluorine-containing amines, amine oxides, alkyl amines and alkyl betaines.

**51.** A dentifrice composition according to Claim 50, characterised in that the organic amino compound is cetyl amine hydrofluoride, bis-(hydroxyethyl)-aminopropyl N-hydroxyethyl octadecylamine dihydrofluoride, an amine oxide of formula

$$R(CH_3)_2\,N \rightarrow O,$$

an alkyl betaine of formula $R\text{-}N^+(CH_3)_2\text{-}CH_2\text{-}COO^-$, an alkylamidoalkyl betaine of formula $R\text{-}CO\text{-}NH_2\text{-}(CH_2)_3\text{-}N^+(CH_3)_2\text{-}CH_2\text{-}COO^-$, in which formulae R represents a straight or branched-chain alkyl radical with 10 to 24 carbon atoms.

**52.** A dentifrice composition according to Claim 49, characterised in that it comprises at least one divalent or higher valency metal cation selected from group 2a, 3a, 4a and 8 of the periodic classification.

**53.** A dentifrice composition according to Claim 52, characterised in that the metal cation is calcium, strontium, barium, aluminium, indium, germanium, tin, lead, manganese, iron, nickel, zinc, titanium, zirconium or palladium.

**54.** A dentifrice composition according to either Claim 52 or Claim 53, characterised in that the metal cation is in the form of the mineral salts, chloride, fluoride, nitrate, phosphate, sulphate or in the form of the organic salts, acetate or citrate.

**55.** A dentifrice composition according to one of claims 52 to 54, characterised in that the metal cation is in the form of zinc citrate, zinc sulphate, strontium chloride, or tin fluoride.

**56.** A dentifrice composition according to Claim 47, characterised in that it comprises chlorhexidine.

**Claims for the following Contracting State : ES**

**1.** A process for the preparation of a silica, this silica having a compatibility with organic amino compounds of at least 30%, and leading to an aqueous suspension whose pH varies as a function of its concentration in the area defined by the two inequations:

$$pH \leq 7.5\text{-}0.7 \log (C) \qquad (Ia)$$

and

$$pH \geq 5.0\text{-}0.5 \log (C) \qquad (Ib)$$

and whose pH varies as a function of its electrical conductivity in the area defined by the two inequations:

$$pH \leq 8.5\text{-}0.4 \log (D) \qquad (IIa)$$

and

$$pH \geq 7.0\text{-}0.6 \log (D) \qquad (IIb)$$

- in the inequations (Ia) and (Ib),
  . (C) represents the weight concentration of the aqueous silica suspension expressed in % $SiO_2$,
- in the inequations (IIa) and (IIb),
  . (D) represents the electrical conductivity of the aqueous silica suspension expressed in microsiemens.$cm^{-1}$,

characterised in that it consists in reacting a silicate with an acid, thus leading to a suspension or a silica gel, carrying out a first ageing operation at a pH equal to or higher than 6 and equal to or lower than 8.5, then a second ageing operation at a pH equal to or lower than 6.0; in carrying out a third ageing operation at a pH equal to or lower than 5.0; separating the silica, washing it in water until it leads to an aqueous suspension whereof the pH measured on a suspension with 20% $SiO_2$ corresponds to the following equation:

$$pH = d - e \log (D) \qquad (III)$$

in which equation (III)

. e is a constant equal to or higher than 0.6, and equal to or lower than 1.0,
. d is a constant equal to or higher than 7.0, and equal to or less than 8.5,
. (D) represents the electrical conductivity of the aqueous silica suspension expressed in microsiemens.$cm^{-1}$;
. finally, in drying it.

**2.** A process according to Claim 1, characterised in that the silica has a surface chemistry such that its acidity function Ho is at least 4.0.

**3.** A process according to either Claim 1 or Claim 2, characterised in that the silica has a surface chemistry, such that the number of $OH^-$ expressed as $OH^-/nm^2$ is equal to or below 12 and preferably between 6 and 10.

4. A process according to one of Claims 1 to 3, characterised in that the silica has a zero point charge (ZPC) of at least 4, preferably of between 4 and 6.

5. A process according to one of Claims 1 to 4, characterised in that the silica has a compatibility with the organic amino compounds of at least 50%, more particularly of at least 80%.

6. A process according to one of Claims 1 to 5, characterised in that the silica has a compatibility with the organic amino compounds selected from the group formed by fluorine-containing amines, amine oxides, alkyl amines and alkyl betaines.

7. A process according to Claim 6, characterised in that the organic amino compound is cetyl amine hydrofluoride, bis-(hydroxyethyl)-aminopropyl-N-hydroxyethyl-octadecyl amine dihydrofluoride, an amine oxide of formula

$$R(CH_3)_2\,N \rightarrow 0,$$

an alkyl betaine of formula $R\text{-}N^+\,(CH_3)_2\text{-}CH_2\text{-}COO^-$, an alkyl amidoalkyl betaine of formula $R\text{-}CO\text{-}NH_2\text{-}(CH_2)_3\text{-}N^+(CH_3)_2\text{-}CH_2\text{-}COO^-$, in which formulae R represents a straight or branched-chain alkyl radical with 10 to 24 carbon atoms.

8. A process according to Claim 1, characterised in that the silica has a compatibility with the divalent and higher valency metal cations selected from groups 2a, 3a, 4a and 8 of the periodic classification of at least 50%, more particularly of at least 70%.

9. A process according to Claim 8, characterised in that the metal cation is calcium, strontium, barium, aluminium, indium, germanium, tin, lead, manganese, iron, nickel, zinc, titanium, zirconium, palladium.

10. A process according to either Claim 8 or Claim 9, characterised in that the metal cation is in the form of the mineral salts, chloride, fluoride, nitrate, phosphate, sulphate, or in the form of the organic salts, acetate and citrate.

11. A process according to Claim 10, characterised in that the metal cation is in the form of zinc citrate, zinc sulphate, strontium chloride or tin fluoride.

12. A process according to Claim 1, characterised in that the silica also has a compatibility with products of the guanidine type, particularly chlorhexidine, of at least 30%, and, more particularly, of at least 60%.

13. A process according to one of Claims 1 to 12, characterised in that the silica has a content of anions of type $SO_4^{2-}$, $Cl^-$, $NO_3^-$, $PO_3^-$, $CO_3^{2-}$ of at the most $5.10^{-3}$ moles/100 g of silica.

14. A process according to Claim 13, characterised in that the silica has a content of the afore-mentioned anions of at the most $1.10^{-3}$, more particularly of at the most $0.2.10^{-3}$ moles/100 g of silica.

15. A process according to one of Claims 1 to 12, characterised in that the silica has a sulphate content, expressed as $SO_4^{2-}$, of at the most 0.1%, preferably at the most 0.05%, and more particularly at the most 0.01%.

16. A process according to one of Claims 1 to 15, characterised in that the silica has a content of divalent and higher valency metal cations of at the most 1000 ppm.

17. A process according to Claim 16, characterised in that the aluminium content is at the most 500 ppm, the iron content is at the most 200 ppm, the calcium content is at the most 500 ppm, and, more particularly, at the most 300 ppm.

18. A process according to one of Claims 1 to 17, characterised in that the silica has a carbon content of at the most 50 ppm, and, more particularly, of at the most 10 ppm.

19. A process according to one of Claims 1 to 18, characterised in that the silica has a pH of at the most 8, and, more particularly, of between 6.0 and 7.5.

20. A process according to one of Claims 1 to 19, characterised in that the silica has a BET surface of between 40 and 600 $m^2/g$.

**21.** A process according to one of Claims 1 to 20, characterised in that the silica has a CTAB surface of between 40 and 400 $m^2/g$.

**22.** A process according to one of Claims 1 to 21, characterised in that the silica is of the abrasive type and has a BET surface of between 40 and 300 $m^2/g$.

**23.** A process according to Claim 22, characterised in that the silica has a CTAB surface of between 40 and 100 $m^2/g$.

**24.** A process according to any one of Claims 1 to 21, characterised in that the silica is of the thickening type and has a BET surface of between 120 and 450 $m^2/g$, more particularly of between 120 and 200 $m^2/g$.

**25.** A process according to Claim 24, characterised in that the silica has a CTAB surface of between 120 and 400 $m^2/g$.

**26.** A process according to one of Claims 1 to 21, characterised in that the silica is of the bifunctional type and has a BET surface of between 80 and 200 $m^2/g$.

**27.** A process according to Claim 26, characterised in that the silica has a CTAB surface of between 80 and 200 $m^2/g$.

**28.** A process according to one of Claims 1 to 27, characterised in that the silica has an oil absorption of between 80 and 500 $cm^3/100$ g.

**29.** A process according to one of Claims 1 to 28, characterised in that the silica has an average particle size of between 1 and 10µm.

**30.** A process according to one of Claims 1 to 29, characterised in that the silica has an apparent density of between 0.01 and 0.3.

**31.** A process according to one of Claims 1 to 30, characterised in that the silica is a precipitation silica.

**32.** A process according to one of Claims 1 to 31, characterised in that it consists in preparing the suspension or silica gel by simultaneously adding the silicate and the acid to a sediment which can be water, a colloidal silica dispersion containing between 0 and 150 g/l silica expressed as $SiO_2$, a silicate or a mineral or organic salt, preferably an alkali metal salt.

**33.** A process according to Claim 32, characterised in that the two reagents are added simultaneously in such a way that the pH is kept constant at between 4 and 10, preferably at between 8.5 and 9.5.

**34.** A process according to either Claim 32 or Claim 33, characterised in that the temperature is between 60°C and 95°C.

**35.** A process according to Claim 32, characterised in that the colloidal silica dispersion containing between 20 and 150 g/l of silica is prepared by heating an aqueous silicate solution at between 60 and 95°C, and by adding acid to said aqueous solution until a pH is obtained of between 8.0 and 10.0, preferably of 9.5.

**36.** A process according to one of Claims 1 to 31, characterised in that it consists in preparing a suspension or a silica gel by gradually adding acid to a sediment containing silicate until the desired ageing pH is obtained at a temperature of between 60°C and 95°C.

**37.** A process according to one of Claims 1 to 34, characterised in that a first ageing operation is carried out on the suspension or silica gel at a pH of between 6 and 8.5, preferably of 8.0, at a temperature of between 60°C and 100°C, preferably of 95°C.

**38.** A process according to one of Claims 1 to 37, characterised in that a second ageing operation is carried out on the suspension or silica gel at a pH of between 5 and 6, preferably of 5.5, at a temperature of between 60°C and 100°C, preferably of 95°C.

**39.** A process according to one of Claims 1 to 38, characterised in that a third ageing operation is carried out on the suspension or silica gel, at a pH of between 3 and 5, preferably of 4, at a temperature of between 60°C and 100°C, preferably of 95°C.

**40.** A process according to one of Claims 1 to 31, characterised in that washing is carried out with water or with the aid of an acid solution.

**41.** A process according to one of Claims 1 to 31, characterized in that washing is carried out until the conductivity of the washing filtrate is at the most 200 microsiemens.$cm^{-1}$.

**42.** A process according to Claim 41, characterised in that washing is carried out with water or with the aid of an acid solution.

**43.** A process according to either Claim 40 or Claim 42, characterised in that the afore-mentioned acid solution is a solution of an organic acid, in particular of a complexing acid.

**44.** A process according to Claim 43, characterised in that the afore-mentioned organic acid is selected from the group of carboxylic acids, dicarboxylic acids, aminocarboxylic acids, hydrocarboxylic acids.

**45.** A process according to either Claim 43 or Claim 44, characterised in that the organic acid is selected from the group comprising acetic, gluconic, tartaric, citric, maleic and glyceric acids.

**46.** A dentifrice composition, characterised in that it contains a silica prepared using the process according to one of Claims 1 to 45.

**47.** A dentifrice composition according to Claim 46, characterised in that it comprises at least one element selected from the group comprising fluorine, phosphates, guanidines.

**48.** A dentifrice composition according to Claim 46, characterised in that it comprises at least one element selected from the group comprising organic amino compounds and divalent and higher valency cations.

**49.** A dentifrice composition according to Claim 46, characterised in that it comprises at least one organic amino compound selected from the group formed by fluorine-containing amines, amine oxides, alkyl amines and alkyl betaines.

**50.** A dentifrice composition according to Claim 49, characterised in that the organic amino compound is cetyl amine hydrofluoride, bis-(hydroxyethyl)-aminopropyl N-hydroxyethyl octadecylamine dihydrofluoride, an amine oxide of formula

$$R(CH_3)_2\,N \rightarrow O,$$

an alkyl betaine of formula $R\text{-}N^+(CH_3)_2\text{-}CH_2\text{-}COO^-$, an alkylamidoalkyl betaine of formula $R\text{-}CO\text{-}NH_2\text{-}(CH_2)_3\text{-}N^+(CH_3)_2\text{-}CH_2\text{-}COO^-$, in which formulae R represents a straight or branched-chain alkyl radical with 10 to 24 carbon atoms.

**51.** A dentifrice composition according to Claim 46, characterised in that it comprises at least one divalent or higher valency metal cation selected from group 2a, 3a, 4a and 8 of the periodic classification.

**52.** A dentifrice composition according to Claim 51, characterised in that the metal cation is calcium, strontium, barium, aluminium, indium, germanium, tin, lead, manganese, iron, nickel, zinc, titanium, zirconium or palladium.

**53.** A dentifrice composition according to either Claim 51 or Claim 52, characterised in that the metal cation is in the form of the mineral salts, chloride, fluoride, nitrate, phosphate, sulphate or in the form of the organic salts, acetate or citrate.

**54.** A dentifrice composition according to one of claims 51 to 53, characterised in that the metal cation is in the form of zinc citrate, zinc sulphate, strontium chloride, or tin fluoride.

**55.** A dentifrice composition according to Claim 46, characterised in that it comprises chlorhexidine.

## Patentansprüche

**Patentansprüche für folgende Vertragsstaaten : AT, BE, CH, DE, DK, FR, GB, GR, IT, LI, LU, NL, SE**

**1.** Kieselsäure, dadurch gekennzeichnet, daß sie eine Verträglichkeit mit den organischen Aminverbindungen von mindestens 30 % aufweist und zu einer wäßrigen Suspension führt, deren pH-Wert gemäß ihrer Konzentration in dem durch die zwei Ungleichungen:

$$pH \leq 7{,}5 - 0{,}7 \log (C) \qquad (Ia)$$

und

$$pH \geq 5{,}0 - 0{,}5 \log (C) \qquad (Ib)$$

bestimmten Bereich schwankt und wobei der pH-Wert gemäß ihrer elektrischen Leitfähigkeit in dem durch die zwei Ungleichungen:

$$pH \leq 8{,}5 - 0{,}4 \log (D) \qquad (IIa)$$

und

$$pH \geq 70 - 0{,}6 \log (D) \qquad (IIb)$$

bestimmten Bereich schwankt, wobei in den Ungleichungen (Ia) und (Ib)

(C) die Gewichtskonzentration der wäßrigen Suspension der Kieselsäure ausgedruckt in % $SiO_2$ und
(D) die elektrische Leitfähigkeit der wäßrigen Kieselsäuresuspension ausgedrückt in Mikrosiemens $cm^{-1}$ darstellen.

**2.** Kieselsäure nach Anspruch 1, dadurch gekennzeichnet, daß sie eine solche Oberflächenchemie aufweist, daß ihre Säurefunktion Ho mindestens 4,0 beträgt.

**3.** Kieselsäure nach einem der Ansprüche 1 und 2, dadurch gekennzeichnet daß sie eine solche Oberflächenchemie aufweist, daß die Anzahl von $OH^-$ausgedrückt in $OH^-/nm^2$ kleiner oder gleich 12, vorzugsweise zwischen 6 und 10 ist.

**4.** Kieselsäure nach einem der Ansprüche 1 bis 3, dadurch gekennzeichnet, daß sie einen Ladungspunkt Null (PZC) von mindestens 4, vorzugsweise zwischen 4 und 6 aufweist.

**5.** Kieselsäure nach einem der Ansprüche 1 bis 4, dadurch gekennzeichnet, daß sie eine Verträglichkeit mit den organischen Aminverbindungen von mindestens 50 %, insbesondere von mindestens 80 % aufweist.

**6.** Kieselsäure nach einem der Ansprüche 1 bis 5, dadurch gekennzeichnet, daß sie eine Verträglichkeit mit den organischen Aminverbindungen aus der Gruppe von Fluoraminen, Aminoxiden, Alkylaminen und Alkylbetainen aufweist.

**7.** Kieselsäure nach Anspruch 6, dadurch gekennzeichnet, daß die organische Aminverbindung Cetylamin-hydrofluorid, Bis-(hydroxyethyl)-aminopropyl-N-hydroxyethyl-octadecylamin-dihydrofluorid, ein Aminoxid der Formel

$$R(CH_3)_2N \rightarrow O,$$

ein Alkylbetain der Formel $R\text{-}N^+(CH_3)_2\text{-}CH_2\text{-}COO^-$, ein Alkylamidoalkylbetain der Formel $R\text{-}CO\text{-}NH_2\text{-}(CH_2)_3\text{-}N^+(CH_3)_2\text{-}CH_2\text{-}COO^-$ ist, wobei in den genannten Formeln R einen geradkettigen oder verzweigten Alkylrest mit 10 bis 24 Kohlenstoffatomen bedeutet.

**8.** Kieselsäure nach Anspruch 1, dadurch gekennzeichnet, daß sie eine Verträglichkeit mit den zwei- und mehrwertigen Metallkationen, ausgewählt aus den Gruppen 2a, 3a, 4a und 8 des periodischen Systems, von mindestens 50 %, insbesondere von mindestens 70 % aufweist.

9. Kieselsäure nach Anspruch 8, dadurch gekennzeichnet, daß das Metallkation Calcium, Strontium, Barium, Aluminium, Indium, Germanium, Zinn, Blei, Mangan, Eisen, Nickel, Zink, Titan, Zirkonium oder Palladium ist.

10. Kieselsäure nach einem der Ansprüche 8 und 9, dadurch gekennzeichnet, daß das Metallkation in Form von anorganischen Salzen als Chlorid, Fluorid, Nitrat, Phosphat, Sulfat oder in Form von organischen Salzen als Acetat, Citrat vorliegt.

11. Kieselsäure nach Anspruch 10, dadurch gekennzeichnet, daß das Metallkation als Zinkcitrat, Zinksulfat, Strontiumchlorid oder Zinnfluorid vorliegt.

12. Kieselsäure nach Anspruch 1, dadurch gekennzeichnet, daß sie außerdem eine Verträglichkeit mit den Verbindungen vom Typ Guanidin, besonders Chlorhexidin von mindestens 30 %, insbesondere von mindestens 60 % aufweist.

13. Kieselsäure nach einem der Ansprüche 1 bis 12, dadurch gekennzeichnet, daß sie einen Aniongehalt vom Typ $SO_4^{2-}$, $Cl^-$, $NO_3^-$, $PO_3^-$, $CO_3^{2-}$ von höchstens $5 \cdot 10^{-3}$ Molen pro 100 g Kieselsäure aufweist.

14. Kieselsäure nach Anspruch 13, dadurch gekennzeichnet, daß sie einen Aniongehalt von $1 \cdot 10^{-3}$, insbesondere von höchstens $0{,}2 \cdot 10^{-3}$ Molen pro 100 g Kieselsäure aufweist.

15. Kieselsäure nach einem der Ansprüche 1 bis 12, dadurch gekennzeichnet, daß sie einen Sulfatgehalt als $SO_4^{2-}$ von höchstens 0,1 %, vorzugsweise von höchstens 0,05 % und insbesondere von höchstens 0,01 % aufweist.

16. Kieselsäure nach einem der Ansprüche 1 bis 15, dadurch gekennzeichnet, daß sie einen Gehalt von zwei- und mehrwertigen Metallkationen von höchstens 1000 ppm aufweist.

17. Kieselsäure nach Anspruch 16, dadurch gekennzeichnet, daß der Aluminiumgehalt höchstens 500 ppm, der Eisengehalt höchstens 200 ppm, der Calciumgehalt höchstens 500 ppm und insbesondere höchstens 300 ppm ist.

18. Kieselsäure nach einem der Ansprüche 1 bis 17, dadurch gekennzeichnet, daß sie einen Kohlenstoffgehalt von höchstens 50 ppm und insbesondere von höchstens 10 ppm aufweist.

19. Kieselsäure nach einem der Ansprüche 1 bis 18, dadurch gekennzeichnet, daß sie einen pH-Wert von höchstens 8 und insbesondere zwischen 6 und 7,5 aufweist.

20. Kieselsäure nach einem der Ansprüche 1 bis 19, dadurch gekennzeichnet, daß sie eine BET-Oberfläche zwischen 40 und 600 $m^2/g$ aufweist.

21. Kieselsäure nach einem der Ansprüche 1 bis 20, dadurch gekennzeichnet, daß sie eine CTAB-Oberfläche zwischen 40 und 400 $m^2/g$ aufweist.

22. Kieselsäure nach einem der Ansprüche 1 bis 21 vom Typ Schleifmittel, dadurch gekennzeichnet, daß sie eine BET-Oberfläche zwischen 40 und 300 $m^2/g$ aufweist.

23. Kieselsäure nach Anspruch 22, dadurch gekennzeichnet, daß sie eine CTAB-Oberfläche zwischen 40 und 100 $m^2/g$ aufweist.

24. Kieselsäure nach einem der Ansprüche 1 bis 21 vom Typ Verdickungsmittel, dadurch gekennzeichnet, daß sie eine BET-Oberfläche zwischen 120 und 450 $m^2/g$, insbesondere 120 und 200 $m^2/g$ aufweist.

25. Kieselsäure nach Anspruch 24, dadurch gekennzeichnet, daß sie eine CTAB-Oberfläche zwischen 120 und 400 $m^2/g$ aufweist.

26. Kieselsäure nach einem der Ansprüche 1 bis 21 vom bifunktionellen Typ, dadurch gekennzeichnet, daß sie eine BET-Oberfläche zwischen 80 und 200 $m^2/g$ aufweist.

27. Kieselsäure nach Anspruch 26, dadurch gekennzeichnet, daß sie eine CTAB-Oberfläche zwischen 80 und 200 $m^2/g$ aufweist.

**28.** Kieselsäure nach einem der Ansprüche 1 bis 27, dadurch gekennzeichnet, daß sie eine Ölaufnahme zwischen 80 und 500 $cm^3$/100 g aufweist.

**29.** Kieselsäure nach einem der Ansprüche 1 bis 28, dadurch gekennzeichnet, daß sie eine mittlere Teilchengröße zwischen 1 und 10 µm aufweist.

**30.** Kieselsäure nach einem der Ansprüche 1 bis 29, dadurch gekennzeichnet, daß sie eine scheinbare Dichte zwischen 0,01 und 0,3 aufweist.

**31.** Kieselsäure nach einem der Ansprüche 1 bis 30, dadurch gekennzeichnet, daß es sich um eine Fällungskieselsäure handelt.

**32.** Verfahren zur Herstellung einer in den Ansprüchen 1 bis 31 beschriebenen Kieselsäure, dadurch gekennzeichnet, daß es darin besteht, daß man ein Silikat mit einer Säure zur Bildung einer Kieselsäuresuspension oder eines Kieselgels reagieren läßt, eine erste Reifung bei einem pH-Wert größer oder gleich 6 und kleiner oder gleich 8,5 ausführt, dann eine zweite Reifung bei einem pH-Wert kleiner oder gleich 6,0 ausführt, eine dritte Reifung bei einem pH-Wert kleiner oder gleich 5,0 ausführt, die Kieselsäure abtrennt und einer Wäsche mit Wasser bis zu einer wäßrigen Suspension, deren pH-Wert, gemessen an einer Suspension von 20 % $SiO_2$, der folgenden Gleichung

$$pH = d - e \log (D) \quad \text{(III)}$$

entspricht, wobei in der Gleichung (III)

- e eine Konstante größer oder gleich 0,6 und kleiner oder gleich 1,0,
- d eine Konstante größer oder gleich 7,0 und kleiner oder gleich 8,5,
- D die elektrische Leitfähigkeit der wäßrigen Kieselsäuresuspension bemessen in Mikrosiemens $cm^{-1}$ bedeuten,

unterwirft und schließlich trocknet.

**33.** Verfahren nach Anspruch 32, dadurch gekennzeichnet, daß man die Kieselsäuresuspension oder das Kieselgel durch gleichzeitige Zugabe des Silikates und der Säure in einer Vorlage herstellt, die Wasser, eine kolloidale Kieselsäuredispersion enthaltend 0 bis 150 g/l Kieselsäure in Form von $SiO_2$, ein Silikat oder ein anorganisches oder organisches Salz, vorzugsweise eines Alkalimetalls sein kann.

**34.** Verfahren nach Anspruch 33, dadurch gekennzeichnet, daß die Zugabe der zwei Reagenzien in der Weise gleichzeitig ausgeführt wird, daß der pH-Wert konstant zwischen 4 und 10, vorzugsweise zwischen 8,5 und 9,5 gehalten wird.

**35.** Verfahren nach einem der Ansprüche 33 und 34, dadurch gekennzeichnet, daß die Temperatur im Bereich zwischen 60 °C und 95 °C liegt.

**36.** Verfahren nach Anspruch 33, dadurch gekennzeichnet, daß man die 20 bis 150 g/l Kieselsäure enthaltende kolloidale Kieselsäuredispersion herstellt, indem man eine wäßrige Silikatlösung auf 60 °C bis 95 °C erhitzt und die Säure der genannten wäßrigen Lösung bis zum Erhalt eines pH-Wertes zwischen 8,0 und 10,0, vorzugsweise gleich 9,5 zugibt.

**37.** Verfahren nach Anspruch 32, dadurch gekennzeichnet, daß es darin besteht, daß man eine Kieselsäuresuspension oder ein Kieselgel herstellt, indem man die Säure allmählich in die das Silikat enthaltende Vorlage bis zum Erhalt des für die Reifung erwünschten pH-Wertes bei einer Temperatur im Bereich zwischen 60 °C und 95 °C hinzufügt.

**38.** Verfahren nach einem der Ansprüche 32 bis 35, dadurch gekennzeichnet, daß man eine erste Reifung der Kieselsäuresuspension oder des Kieselgels bei einem pH-Wert zwischen 6 und 8,5, vorzugsweise bei 8,0, und bei einer Temperatur zwischen 60 °C und 100 °C, vorzugsweise bei 95 °C ausführt.

**39.** Verfahren nach einem der Ansprüche 32 bis 38, dadurch gekennzeichnet, daß man eine zweite Reifung der Kieselsäuresuspension oder des Kieselgels bei einem pH-Wert zwischen 5 und 6, vorzugsweise bei 5,5, und bei einer Temperatur zwischen 60 °C und 100 °C, vorzugsweise 95 °C durchführt.

**40.** Verfahren nach einem der Ansprüche 32 bis 39, dadurch gekennzeichnet, daß man eine dritte Reifung der Kieselsäuresuspension oder des Kieselgels bei einem pH-Wert zwischen 3 und 5, vorzugsweise gleich 4, und bei einer Temperatur im Bereich zwischen 60 °C und 100 °C, vorzugsweise bei 95 °C ausführt.

**41.** Verfahren nach Anspruch 32, dadurch gekennzeichnet, daß man eine Wäsche mit Wasser oder mit Hilfe einer sauren Lösung durchfuhrt.

**42.** Verfahren nach Anspruch 32, dadurch gekennzeichnet, daß man eine Wäsche bis zum Erhalt einer Leitfähigkeit des Waschfiltrates von höchstens 200 Mikrosiemens $cm^{-1}$ durchführt.

**43.** Verfahren nach Anspruch 42, dadurch gekennzeichnet, daß man eine Wäsche mit Wasser oder mit Hilfe einer sauren Lösung durchführt.

**44.** Verfahren nach einem der Ansprüche 41 und 43, dadurch gekennzeichnet, daß die erwähnte saure Lösung eine Lösung einer organischen Säure, insbesondere einer komplexbildenden Säure ist.

**45.** Verfahren nach Anspruch 44, dadurch gekennzeichnet, daß die erwähnte saure Lösung aus der Gruppe der Carbonsäuren, Dicarbonsäuren, Aminocarbonsäuren und Hydrocarbonsäuren ausgewählt ist.

**46.** Verfahren nach einem der Ansprüche 44 und 45, dadurch gekennzeichnet, daß die organische Säure aus der Gruppe umfassend Essigsäure, Gluconsäure, Weinsäure, Citronensäure, Maleinsäure und Glycerinsäure ausgewählt ist.

**47.** Zahnpflegemittelzusammensetzung, dadurch gekennzeichnet, daß sie eine Kieselsäure nach einem der Ansprüche 1 bis 31 oder eine gemäß dem Verfahren nach einem der Ansprüche 32 bis 46 hergestellte Kieselsäure enthält.

**48.** Zahnpflegemittelzusammensetzung nach Anspruch 47, dadurch gekennzeichnet, daß sie mindestens ein Element ausgewählt aus der Gruppe von Fluor, Phosphate, Guanidine enthält.

**49.** Zahnpflegemittelzusammensetzung nach Anspruch 47, dadurch gekennzeichnet, daß sie mindestens ein Element ausgewählt aus der Gruppe von organischen Aminverbindungen und zwei- und mehrwertigen Kationen enthält.

**50.** Zahnpflegemittelzusammensetzung nach Anspruch 47, dadurch gekennzeichnet, daß sie mindestens eine organische Aminverbindung ausgewählt aus der Gruppe der fluorierten Amine, Aminoxide, Alkylamine und Alkylbetaine enthält.

**51.** Zahnpflegemittelzusammensetzung nach Anspruch 50, dadurch gekennzeichnet, daß die organische Aminverbindung Cetylamin-hydrofluorid, Bis-(hydroxyethyl)-aminopropyl-N-hydroxyethyl-octadecylamin-dihydrofluorid, ein Aminoxid der Formel

$$R(CH_3)_2N \rightarrow O,$$

ein Alkylbetain der Formel $R\text{-}N^+(CH_3)_2\text{-}CH_2\text{-}COO^-$, ein Alkylamidoalkylbetain der Formel $R\text{-}CO\text{-}NH_2\text{-}(CH_2)_3\text{-}N^+(CH_3)_2\text{-}CH_2\text{-}COO^-$ ist, wobei in den genannten Formeln R einen geradkettigen oder verzweigten Alkylrest mit 10 bis 24 Kohlenstoffatomen darstellt.

**52.** Zahnpflegemittelzusammensetzung nach Anspruch 49, dadurch gekennzeichnet, daß sie mindestens ein zwei- oder mehrwertiges Metallkation ausgewählt aus den Gruppen 2a, 3a, 4a und 8 des periodischen Systems enthält.

**53.** Zahnpflegemittelzusammensetzung nach Anspruch 52, dadurch gekennzeichnet, daß das Metallkation Calcium, Strontium, Barium, Aluminium, Indium, Germanium, Zinn, Blei, Mangan, Eisen, Nickel, Zink, Titan, Zirkonium oder Palladium ist.

**54.** Zahnpflegemittelzusammensetzung nach einem der Ansprüche 52 und 53, dadurch gekennzeichnet, daß das Metallkation in Form von anorganischen Salzen als Chlorid, Fluorid, Nitrat, Phosphat, Sulfat oder in Form von organischen Salzen als Acetat, Citrat vorliegt.

**55.** Zahnpflegemittelzusammensetzung nach einem der Ansprüche 52 bis 54, dadurch gekennzeichnet, daß das

Metallkation als Zinkcitrat, Zinksulfat, Strontiumchlorid oder Zinnfluorid vorliegt.

**56.** Zahnpflegemittelzusammensetzung nach Anspruch 47, dadurch gekennzeichnet, daß sie Chlorhexidin enthält.

**Patentansprüche für folgenden Vertragsstaat : ES**

**1.** Verfahren zur Herstellung einer Kieselsäure, dadurch gekennzeichnet, daß sie eine Verträglichkeit mit den organischen Aminverbindungen von mindestens 30 % aufweist und zu einer wäßrigen Suspension führt, deren pH-Wert gemäß ihrer Konzentration im durch die zwei Ungleichungen:

$$pH \leq 7{,}5 - 0{,}7 \log (C) \qquad (Ia)$$

und

$$pH \geq 5{,}0 - 0{,}5 \log (C) \qquad (Ib)$$

bestimmten Bereich schwankt und wobei der pH-Wert gemäß ihrer elektrischen Leitfähigkeit im durch die zwei Ungleichungen:

$$pH \leq 8{,}5 - 0{,}4 \log (D) \qquad (IIa)$$

und

$$pH \geq 7{,}0 - 0{,}6 \log (D) \qquad (IIb)$$

bestimmten Bereich schwankt, wobei in den Ungleichungen (Ia) und (Ib)

(C) die Gewichtskonzentration der wäßrigen Suspension der Kieselsäure ausgedrückt in % von $SiO_2$ und
(D) die elektrische Leitfähigkeit der wäßrigen Kieselsäuresuspension ausgedrückt in Mikrosiemens $cm^{-1}$ darstellen, dadurch gekennzeichnet,

daß es darin besteht, daß man ein Silikat mit einer Säure zur Bildung einer Kieselsäuresuspension oder eines Kieselgels reagieren läßt, eine erste Reifung bei einem pH-Wert größer oder gleich 6 und kleiner oder gleich 8,5 ausführt, dann eine zweite Reifung bei einem pH-Wert kleiner oder gleich 6,0 ausführt, eine dritte Reifung bei einem pH-Wert kleiner oder gleich 5,0 ausführt, die Kieselsäure abtrennt, einer Wäsche mit Wasser bis zu einer wäßrigen Suspension, deren pH-Wert, gemessen an einer Suspension von 20 % $SiO_2$, der folgenden Gleichung

$$pH = d - e \log (D) \qquad (III)$$

entspricht, wobei in der Gleichung (III)

- e eine Konstante größer oder gleich 0,6 und kleiner oder gleich 1,0,
- d eine Konstante größer oder gleich 7,0 und kleiner oder gleich 8,5,
- D die elektrische Leitfähigkeit der wäßrigen Kieselsäuresuspension bemessen in Mikrosiemens $cm^{-1}$ bedeuten, unterwirft und schließlich trocknet.

**2.** Verfahren nach Anspruch 1, dadurch gekennzeichnet, daß die Kieselsäure eine solche Oberflächenchemie aufweist, daß ihre Säurefunktion Ho mindestens 4,0 beträgt.

**3.** Verfahren nach einem der Ansprüche 1 und 2, dadurch gekennzeichnet, daß die Kieselsäure eine solche Oberflächenchemie aufweist, daß die Anzahl von $OH^-$ ausgedrückt in $OH^-/nm^2$ kleiner oder gleich 12, vorzugsweise zwischen 6 und 10 ist.

**4.** Verfahren nach einem der Ansprüche 1 bis 3, dadurch gekennzeichnet, daß die Kieselsäure einen Ladungspunkt Null (PZC) von mindestens 4, vorzugsweise zwischen 4 und 6 aufweist.

**5.** Verfahren nach einem der Ansprüche 1 bis 4, dadurch gekennzeichnet, daß die Kieselsäure eine Verträglichkeit mit den organischen Aminverbindungen von mindestens 50 %, insbesondere von mindestens 80 % aufweist.

6. Verfahren nach einem der Ansprüche 1 bis 5, dadurch gekennzeichnet, daß die Kieselsäure eine Verträglichkeit mit den organischen Aminverbindungen aus der Gruppe von Fluoraminen, Aminoxiden, Alkylaminen und Alkylbetainen aufweist.

7. Verfahren nach Anspruch 6, dadurch gekennzeichnet, daß die organische Aminverbindung Cetylamin-hydrofluorid, Bis-(hydroxyethyl)-aminopropyl-N-hydroxyethyl-octadecylamin-dihydrofluorid, ein Aminoxid der Formel

$$R(CH_3)_2N \rightarrow O,$$

ein Alkylbetain der Formel $R\text{-}N^+(CH_3)_2\text{-}CH_2\text{-}COO^-$, ein Alkylamidoalkylbetain der Formel $R\text{-}CO\text{-}NH_2\text{-}(CH_2)_3\text{-}N^+(CH_3)_2\text{-}CH_2\text{-}COO^-$ ist, wobei in den genannten Formeln R einen geradkettigen oder verzweigten Alkylrest mit 10 bis 24 Kohlenstoffatomen bedeutet.

8. Verfahren nach Anspruch 1, dadurch gekennzeichnet, daß die Kieselsäure eine Verträglichkeit mit den zwei- und mehrwertigen Metallkationen, ausgewählt aus den Gruppen 2a, 3a, 4a und 8 des periodischen Systems, von mindestens 50 %, insbesondere von mindestens 70 % aufweist.

9. Verfahren nach Anspruch 8, dadurch gekennzeichnet, daß das Metallkation Calcium, Strontium, Barium, Aluminium, Indium, Germanium, Zinn, Blei, Mangan, Eisen, Nickel, Zink, Titan, Zirkonium oder Palladium ist.

10. Verfahren nach einem der Ansprüche 8 und 9, dadurch gekennzeichnet, daß das Metallkation in Form von anorganischen Salzen als Chlorid, Fluorid, Nitrat, Phosphat, Sulfat oder in Form von organischen Salzen als Acetat, Citrat vorliegt.

11. Verfahren nach Anspruch 10, dadurch gekennzeichnet, daß das Metallkation als Zinkcitrat, Zinksulfat, Strontiumchlorid oder Zinnfluorid vorliegt.

12. Verfahren nach Anspruch 1, dadurch gekennzeichnet, daß die Kieselsäure außerdem eine Verträglichkeit mit den Verbindungen vom Typ Guanidin, besonders Chlorhexidin von mindestens 30 %, insbesondere von mindestens 60 % aufweist.

13. Verfahren nach einem der Ansprüche 1 bis 12, dadurch gekennzeichnet, daß die Kieselsäure einen Aniongehalt vom Typ $SO_4^{2-}$, $Cl^-$, $NO_3^-$, $PO_3^-$, $CO_3^{2-}$ von höchstens $5 \cdot 10^{-3}$ Molen pro 100 g Kieselsäure aufweist.

14. Verfahren nach Anspruch 13, dadurch gekennzeichnet, daß die Kieselsäure einen Aniongehalt von $1 \cdot 10^{-3}$, insbesondere von höchstens $0{,}2 \cdot 10^{-3}$ Molen pro 100 g Kieselsäure aufweist.

15. Verfahren nach einem der Ansprüche 1 bis 12, dadurch gekennzeichnet, daß die Kieselsäure einen Sulfatgehalt als $SO_4^{2-}$ von höchstens 0,1 %, vorzugsweise von höchstens 0,05 % und insbesondere von höchstens 0,01 % aufweist.

16. Verfahren nach einem der Ansprüche 1 bis 15, dadurch gekennzeichnet, daß die Kieselsäure einen Gehalt von zwei- und mehrwertigen Metallkationen von höchstens 1000 ppm aufweist.

17. Verfahren nach Anspruch 16, dadurch gekennzeichnet, daß der Aluminiumgehalt höchstens 500 ppm, der Eisengehalt höchstens 200 ppm, der Calciumgehalt höchstens 500 ppm und insbesondere höchstens 300 ppm ist.

18. Verfahren nach einem der Ansprüche 1 bis 17, dadurch gekennzeichnet, daß die Kieselsäure einen Kohlenstoffgehalt von höchstens 50 ppm und insbesondere von höchstens 10 ppm aufweist.

19. Verfahren nach einem der Ansprüche 1 bis 18, dadurch gekennzeichnet, daß die Kieselsäure einen pH-Wert von höchstens 8 und insbesondere zwischen 6 und 7,5 aufweist.

20. Verfahren nach einem der Ansprüche 1 bis 19, dadurch gekennzeichnet, daß die Kieselsäure eine BET-Oberfläche zwischen 40 und 600 $m^2/g$ aufweist.

21. Verfahren nach einem der Ansprüche 1 bis 20, dadurch gekennzeichnet, daß die Kieselsäure eine CTAB-Oberfläche zwischen 40 und 400 $m^2/g$ aufweist.

22. Verfahren nach einem der Ansprüche 1 bis 21, dadurch gekennzeichnet, daß die Kieselsäure vom Typ Schleifmittel eine BET-Oberfläche zwischen 40 und 300 $m^2/g$ aufweist.

23. Verfahren nach Anspruch 22, dadurch gekennzeichnet, daß die Kieselsäure eine CTAB-Oberfläche zwischen 40 und 100 $m^2/g$ aufweist.

24. Verfahren nach einem der Ansprüche 1 bis 21, dadurch gekennzeichnet, daß die Kieselsäure vom Typ Verdikkungsmittel eine BET-Oberfläche zwischen 120 und 450 $m^2/g$, insbesondere 120 und 200 $m^2/g$ aufweist.

25. Verfahren nach Anspruch 24, dadurch gekennzeichnet, daß die Kieselsäure eine CTAB-Oberfläche zwischen 120 und 400 $m^2/g$ aufweist.

26. Verfahren nach einem der Ansprüche 1 bis 21, dadurch gekennzeichnet, daß die Kieselsäure vom bifunktionellen Typ eine BET-Oberfläche zwischen 80 und 200 $m^2/g$ aufweist.

27. Verfahren nach Anspruch 26, dadurch gekennzeichnet, daß die Kieselsäure eine CTAB-Oberfläche zwischen 80 und 200 $m^2/g$ aufweist.

28. Verfahren nach einem der Ansprüche 1 bis 27, dadurch gekennzeichnet, daß die Kieselsäure eine Ölaufnahme zwischen 80 und 500 $cm^3/100$ g aufweist.

29. Verfahren nach einem der Ansprüche 1 bis 28, dadurch gekennzeichnet, daß die Kieselsäure eine mittlere Teilchengröße zwischen 1 und 10 µm aufweist.

30. Verfahren nach einem der Ansprüche 1 bis 29, dadurch gekennzeichnet, daß die Kieselsäure eine scheinbare Dichte zwischen 0,01 und 0,3 aufweist.

31. Verfahren nach einem der Ansprüche 1 bis 30, dadurch gekennzeichnet, daß die Kieselsäure eine Fällungskieselsäure ist.

32. Verfahren nach einem der Ansprüche 1 bis 31, dadurch gekennzeichnet, daß es darin besteht, daß man die Kieselsäuresuspension oder das Kieselgel durch gleichzeitige Zugabe des Silikates und der Säure in einer Vorlage herstellt, die Wasser, eine kolloidale Kieselsäuredispersion enthaltend 0 bis 150 g/l Kieselsäure in Form von $SiO_2$, ein Silikat oder ein anorganisches oder organisches Salz, vorzugsweise eines Alkalimetalls sein kann.

33. Verfahren nach Anspruch 32, dadurch gekennzeichnet, daß die Zugabe der zwei Reagenzien in der Weise gleichzeitig ausgeführt wird, daß der pH-Wert konstant zwischen 4 und 10, vorzugsweise zwischen 8,5 und 9,5 gehalten wird.

34. Verfahren nach einem der Ansprüche 32 und 33, dadurch gekennzeichnet, daß die Temperatur im Bereich zwischen 60 °C und 95 °C liegt.

35. Verfahren nach Anspruch 32, dadurch gekennzeichnet, daß man die 20 bis 150 g/l Kieselsäure enthaltende kolloidale Kieselsäuredispersion herstellt, indem man eine wäßrige Silikatlösung auf 60 °C bis 95 °C erhitzt und die Säure der genannten wäßrigen Lösung bis zum Erhalt eines pH-Wertes zwischen 8,0 und 10,0, vorzugsweise gleich 9,5 zugibt.

36. Verfahren nach einem der Ansprüche 1 bis 31, dadurch gekennzeichnet, daß es darin besteht, daß man eine Kieselsäuresuspension oder ein Kieselgel herstellt, indem man die Säure allmählich in die das Silikat enthaltende Vorlage bis zum Erhalt des für die Reifung erwünschten pH-Wertes bei einer Temperatur im Bereich zwischen 60 °C und 95 °C hinzufügt.

37. Verfahren nach einem der Ansprüche 1 bis 34, dadurch gekennzeichnet, daß man eine erste Reifung der Kieselsäuresuspension oder des Kieselgels bei einem pH-Wert zwischen 6 und 8,5, vorzugsweise bei 8,0, und bei einer Temperatur im Bereich zwischen 60 °C und 100 °C, vorzugsweise bei 95° C ausführt.

38. Verfahren nach einem der Ansprüche 1 bis 37, dadurch gekennzeichnet, daß man eine zweite Reifung der Kieselsäuresuspension oder des Kieselgels bei einem pH-Wert zwischen 5 und 6, vorzugsweise bei 5,5, und bei einer Temperatur zwischen 60 °C und 100 °C, vorzugsweise bei 95 °C durchführt.

**39.** Verfahren nach einem der Ansprüche 1 bis 38, dadurch gekennzeichnet, daß man eine dritte Reifung der Kieselsäuresuspension oder des Kieselgels bei einem pH-Wert zwischen 3 und 5, vorzugsweise bei 4, bei einer Temperatur im Bereich zwischen 60 °C und 100 °C, vorzugsweise bei 95 °C ausführt.

**40.** Verfahren nach einem der Ansprüche 1 bis 31, dadurch gekennzeichnet, daß man eine Wäsche mit Wasser oder mit Hilfe einer sauren Lösung durchführt.

**41.** Verfahren nach einem der Ansprüche 1 bis 31, dadurch gekennzeichnet, daß man eine Wäsche bis zum Erhalt einer Leitfähigkeit des Waschfiltrates von höchstens 200 Mikrosiemens $cm^{-1}$ durchführt.

**42.** Verfahren nach Anspruch 41, dadurch gekennzeichnet, daß man eine Wäsche mit Wasser oder mit Hilfe einer sauren Lösung durchführt.

**43.** Verfahren nach einem der Ansprüche 40 und 42, dadurch gekennzeichnet, daß die erwähnte saure Lösung eine Lösung einer organischen Säure, insbesondere einer komplexbildenden Säure ist.

**44.** Verfahren nach Anspruch 43, dadurch gekennzeichnet, daß die erwähnte saure Lösung aus der Gruppe der Carbonsäuren, Dicarbonsäuren, Aminocarbonsäuren und Hydrocarbonsäuren ausgewählt ist.

**45.** Verfahren nach einem der Ansprüche 43 und 44, dadurch gekennzeichnet, daß die organische Säure aus der Gruppe umfassend Essigsäure, Gluconsäure, Weinsäure, Citronensäure, Maleinsäure und Glycerinsäure ausgewählt ist.

**46.** Zahnpflegemittelzusammensetzung, dadurch gekennzeichnet, daß sie eine gemäß dem Verfahren nach einem der Ansprüche 1 bis 45 hergestellte Kieselsäure enthält.

**47.** Zahnpflegemittelzusammensetzung nach Anspruch 46, dadurch gekennzeichnet, daß sie mindestens ein Element ausgewählt aus der Gruppe von Fluor, Phosphate, Guanidine enthält.

**48.** Zahnpflegemittelzusammensetzung nach Anspruch 46, dadurch gekennzeichnet, daß sie mindestens ein Element ausgewählt aus der Gruppe von organischen Aminverbindungen und zwei- und mehrwertigen Kationen enthält.

**49.** Zahnpflegemittelzusammensetzung nach Anspruch 46, dadurch gekennzeichnet, daß sie mindestens eine organische Aminverbindung ausgewählt aus der Gruppe der fluorierten Amine, Aminoxide, Alkylamine und Alkylbetaine enthält.

**50.** Zahnpflegemittelzusammensetzung nach Anspruch 49, dadurch gekennzeichnet, daß die organische Aminverbindung Cetylamin-hydrofluorid, Bis-(hydroxyethyl)-aminopropyl-N-hydroxyethyl-octadecylamin-dihydrofluori d, ein Aminoxid der Formel

$$R(CH_3)_2N \rightarrow O,$$

ein Alkylbetain der Formel $R\text{-}N^+(CH_3)_2\text{-}CH_2\text{-}COO^-$, ein Alkylamidoalkylbetain der Formel $R\text{-}CO\text{-}NH_2\text{-}(CH_2)_3\text{-}N^+(CH_3)_2\text{-}CH_2\text{-}COO^-$ ist, wobei in den genannten Formeln R einen geradkettigen oder verzweigten Alkylrest mit 10 bis 24 Kohlenstoffatomen darstellt.

**51.** Zahnpflegemittelzusammensetzung nach Anspruch 46, dadurch gekennzeichnet, daß sie mindestens ein zwei- oder mehrwertiges Metallkation ausgewählt aus den Gruppen 2a, 3a, 4a und 8 des periodischen Systems enthält.

**52.** Zahnpflegemittelzusammensetzung nach Anspruch 51, dadurch gekennzeichnet, daß das Metallkation Calcium, Strontium, Barium, Aluminium, Indium, Germanium, Zinn, Blei, Mangan, Eisen, Nickel, Zink, Titan, Zirkonium oder Palladium ist.

**53.** Zahnpflegemittelzusammensetzung nach einem der Ansprüche 51 und 52, dadurch gekennzeichnet, daß das Metallkation in Form von anorganischen Salzen als Chlorid, Fluorid, Nitrat, Phosphat, Sulfat oder in Form von organischen Salzen als Acetat, Citrat vorliegt.

**54.** Zahnpflegemittelzusammensetzung nach einem der Ansprüche 51 bis 53, dadurch gekennzeichnet, daß das Metallkation in Form von Zinkcitrat, Zinksulfat, Strontiumchlorid oder Zinnfluorid vorliegt.

**55.** Zahnpflegemittelzusammensetzung nach Anspruch 46, dadurch gekennzeichnet, daß sie Chlorhexidin enthält.